(19) Europäisches Patentamt
European Patent Office
Office européen des brevets

(11) EP 0 714 402 B1

(12) **EUROPEAN PATENT SPECIFICATION**

(45) Date of publication and mention of the grant of the patent:
**15.11.2000 Bulletin 2000/46**

(21) Application number: **94924920.5**

(22) Date of filing: **23.08.1994**

(51) Int. Cl.7: **C07K 1/10**, C07K 1/13, A61K 49/00, A61K 47/48, A61K 9/127

(86) International application number:
**PCT/GB94/01844**

(87) International publication number:
**WO 95/06058 (02.03.1995 Gazette 1995/10)**

(54) **POLYMER MODIFICATION**
MODIFIKATION VON POLYMEREN
MODIFICATION DE POLYMERE

(84) Designated Contracting States:
**AT BE CH DE DK ES FR GB GR IE IT LI LU MC NL PT SE**

(30) Priority: **24.08.1993 GB 9317618**

(43) Date of publication of application:
**05.06.1996 Bulletin 1996/23**

(60) Divisional application:
**00105847.8 / 1 026 171**

(73) Proprietor:
**PolyMASC Pharmaceuticals plc**
**London NW3 2EZ (GB)**

(72) Inventors:
- **FRANCIS, Gillian, Elizabeth**
**Molecular Cell Patho.**
**Rowland Hill Street London NW3 2PF (GB)**
- **FISHER, Derek**
**Molecular Cell Pathology**
**Rowland Hill Street London NW3 2PF (GB)**
- **DELGADO, Cristina**
**Molecular Cell Pathology**
**Rowland Hill Street London NW3 2PF (GB)**
- **MALIK, Farooq**
**Molecular Cell Pathology**
**Rowland Hill Street London NW3 2PF (GB)**

(74) Representative:
**Nachshen, Neil Jacob et al**
**D Young & Co**
**21 New Fetter Lane**
**London EC4A 1DA (GB)**

(56) References cited:
**WO-A-90/04384** **WO-A-90/04606**
**WO-A-90/04650**

- **EXP.HEMATOL., vol.20, 1992 pages 1028 - 35 F.MALIK ET AL. 'Polyethylene Glycol (PEG)-modified Granulocyte-Macrophage Colony-stimulating Factor (GM-CSF) with conserved Biological Activity'**
- **BIOTECHNOL.APPL.BIOCHEM., vol.12, no.2, 1990 pages 119 - 128 C.DELAGO ET AL. 'Coupling of Poly(ethylene glycol) to Albumin under Very Mild Conditions by Activation with Tresyl Chloride: Characterization of the Conjugate by Partitioning in Aqueous Two-Phase Systems'**
- **BIOCHEM.BIOPHYS.ACTA, vol.106, 1991, NETHERLAND pages 77 - 82 J.SENIOR ET AL. 'Influence of surface hydrophilicty of liposomes on their interaction with plasma protein and clearance from the circulation: studies with poly(ethylene glycol)-coated vesicles'**
- **BRITHISH JOURNAL OF HAEMATOLOGY, vol.82, 1992 pages 654 - 663 C.KNÜSLI ET AL. 'Polyethylene glycol (PEG) modification of granulocyte-macrophage colony stimulating factor (GM-CSF) enhances neutrophil priming activity but not colony stimulating activity'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **ANALYTICAL.BIOCHEM., vol.134, 1983 pages 60 - 72 K.NILSSON ET AL. 'High-Performance Liquid Chromatography on Silica-Bound Alcohol Dehydrogenase'**
- **BIOCHEM.SOC.TRANS., vol.16, no.6, 1988 pages 968 - 969 C.DELAGO ET AL. 'Immunoaffinity cell partitioning of erythrocytes'**

Remarks:

The file contains technical information submitted after the application was filed and not included in this specification

## Description

The present invention relates to a process for direct covalent bonding of polymer moieties to target molecules in order to improve or modify the biological properties of the target molecules. The invention also relates to certain products which may be produced by this process and to intermediates useful in the process as well as to methods of use of the products of the process.

Covalent attachment of polyethylene glycol(PEG) and other polymers to proteins is well known to convey many benefits which improve the pharmacological and some physiological properties of proteins (reviewed in Nucci et al (1991) Advanced Drug Delivery Reviews, **6**, 133-151; Zalipsky & Lee (1992) Biomedical Applications of Polyethylene Glycol Chemistry (ed. by J.M. Harris), Plenum, New York; Fuertges & Abuchowski, Journal of Controlled Release, **11**, 139-148 1990).

There are many methods for achieving covalent coupling of polymers like PEG to proteins. All consist of a method of activating the polymer by attachment of a group or groups herein called an "activating moiety" or by coverting a terminal moiety of the polymer into an "activating moiety" and a second step where the polymer couples to the target molecule, usually via a residual portion of the "activating moiety" herein called the "coupling moiety".

Typical targets are proteins which contain one or more reactive groups on which the attachment of the coupling moiety or polymer itself can take place, such as primary and secondary amino groups, thiol groups and aromatic hydroxy groups. Alternatively, the protein molecule has been modified so that it contains suitable groups. Similar techniques are applied in coupling PEG and other polymers to other types of target molecule.

Examples of known techniques are:

Cyanuric Chloride Methods: USP 4 179 337 Davis at al; USP 4 301 144 Iwashita & Ajisaka; USP 4 261 973 Lee & Sehon; EP-A-0 098 110 Hiratani; USP 4 495 285 Shimitzu; USP 4 609 546 Hiratani; WO 86/04145 Tomasi; EP-A-0 210 761 Miyata et al; WO 90/06952 Ishikawa at al; GB 2 238 959A Sehon at al; Abuchowski at al (1977a), Journal of Biological Chemistry, **252**, 3578-3581, Abuchowski at al (1977b), Journal of Biological Chemistry, **252**, 3582-3586.

Succinimidyl Active Ester Methods: Boccu at al (1983) Z. Naturforsch, **38c** 94-99; Abuchowski et al (1984) Cancer Biochem. Biophys., **7**, 175-186; Leonard at al (1984) Tetrahedron, **40**, 1585-1590; USP 4 412 989 Iwashita at al; WO 86/04145 Tomasi; WO 87/00056 Katre & Knauf; EP-A-0 247 860 Katre at al; EP-A-0-260 098 Inada at al; WO 89/06546 Shadle at al; Katre at al (1987) Proc. Natl. Acad. Sci. USA, **84**, 1487-1491.

Carbonyldiimidazole Method: Beauchamp at al (1983) Analytical Biochemistry, **131**, 25-33; EP-A-0 154 432 Sawai et al.

Phenylchloroformate Methods: Veronese et al (1985) Appl. Biochem. Biotechnol., **11**, 141-152; WO 89/06546 Shadle et al; WO 90/15628 Groves et al.

PEG-Succinate Mixed Anhydride Methods: Ahlstedt et al (1983) Int. Arch. Allergy Appl. Immunol., **71**,228-232; Richter and Akerblom (1983) Int. Arch. Allergy Appl. Immunol, **70**, 124-131; Richter & Akerblom (1984) Int. Arch. Allergy Appl. Immunol, **74**, 36-39; Lee and Sehon (1978) Immunol. Rev, **41**, 200-247; USP 4 261 973 Lee and Sehon.

Organic Sulphonyl Halide Methods: USP 4 415 665 Mosbach & Nilsson; Delgado at al (1990) Biotechnology and Applied Biochemistry, **12**, 119-128; WO 90/04650 Francis et al; WO 90/04606 Delgado et al; WO 90/04384 Fisher.

PEG-Aldehyde Methods: Harris et al (1989) Separations Using Aqeous Phase Systems. Applications in Cell Biology and Biotechnology (ed. by D. Fisher and I.A. Sutherland), p.203. Plenum Press, London; Harris et al (1991) Water-Soluble Polymers (ed. by S.W. Shalaby, C.L. McCormick and G.B. Butler), American Chemical Society, Washington, D.C. USP 4 002 531 Royer; EP-A-0-251 304 Minami et al; WO 90/05534 Capon et al.

PEG-Maleimide and Related Methods: Goodson & Katre (1990) Biotechnology, **8**, 343-346.

Phenylglyoxal Method: EP-A-0 340 741 Maeda et al.

Succinimide Carbonate Method: WO 90/13540 Zalipsky; WO 91/07190 Nho et al;

Cyanogen Bromide Method: USP 4 301 144 Iwashita & Ajisaka.

Poly-PEG Maleic Acid Anhydride Method: Yoshimoto et al (1987) Biochemical and Biophysical Research Communications, **148**, 876-882.

The key features of the methods are summarised in Table 1.

**Table 1**

| Method | Coupling Moiety (Boxed) and general structure of adduct | Bond | Co-Product | Recommended Coupling Reaction |
|---|---|---|---|---|
| Cyanuric Chloride | 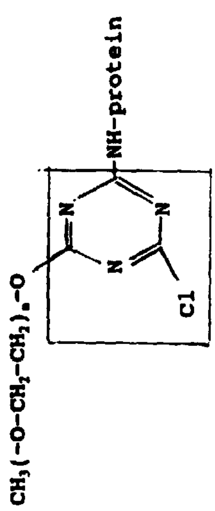 | | HCl | 1h, RT[2],pH 9.2(3)<br>1h, RT, pH 9.8 (4)<br>1h, 37°C, pH10(5) |
| Succinimidyl active ester (succinimidyl succinate) | 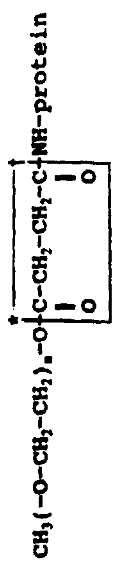 | †amide bond<br>*ester bond | N-hydroxy-succinimide | 30 min, pH 7(6)<br>1h, RT, pH8.25(7)<br>30min, RT, pH9(8) |
| PEG-succinate mixed anhydride | 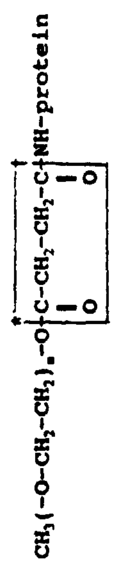 | † amide bond<br>* ester bond | ester plus ester-modified protein | 30 min, RT plus overnight 0°C pH 8.7 (9) |
| Phenylchloro-formates | 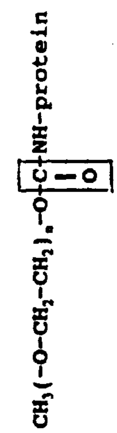 | carbamate | substituted phenol[1] | 1-5h,pH8.3-9.3(10) |
| Carbonyldiimi-dazole | 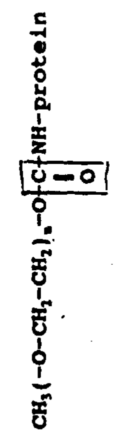 | carbamate | imidazole | 24-72h, 4°C,pH8.5 (11,12) |

| Method | Coupling Moiety (Boxed) and general structure of adduct | Bond | Co-Product | Recommended Coupling Reaction |
|---|---|---|---|---|
| Succinimide carbonate | 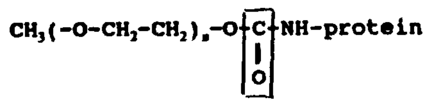 | carbamate | N-hydroxy-succinimide | 30min,pH9.3 (13) |
| Poly(PEG-MA) anhydride | 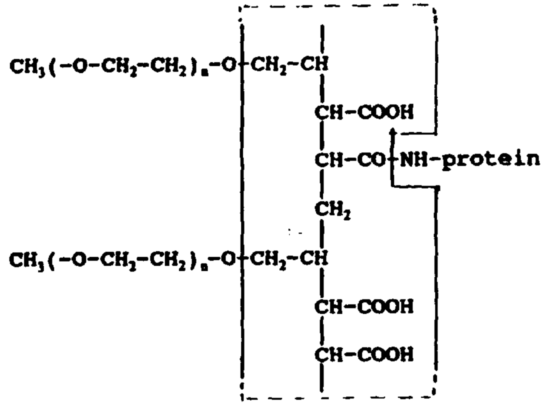 | †amide bond | | 2h,25°C,pH8.5 (14) |
| PEG-maleimide | 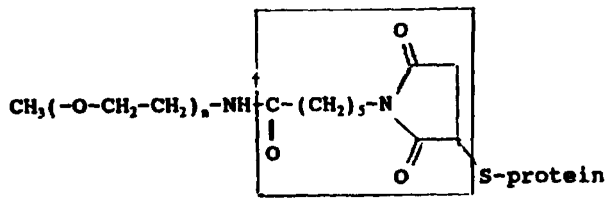 | †amide bond | | several hours,RT, pH5-7 (15)<br>2hr, RT, pH5.5 (16) |

| Method | Coupling Moiety (Boxed) and general structure of adduct | Bond | Co-Product | Recommended Coupling Reaction |
|---|---|---|---|---|
| PEG-acetaldehyde | no coupling moiety present or ethylene oxide unit<br>$CH_3(-O-CH_2-CH_2)_n-O-CH_2-CH_2$ ‡ $NH$-protein | ‡ secondary amine | oxidised cyano-borohydride | Not indicated (17) |
| Sulphonic halogenide | no coupling moiety present<br>$CH_3(-O-CH_2-CH_2)_n$ ‡ $-NH$-protein | ‡ secondary amine | sulphonic acid | 1h,RT,pH 7.5 (18) |
| Phenyl glyoxal | 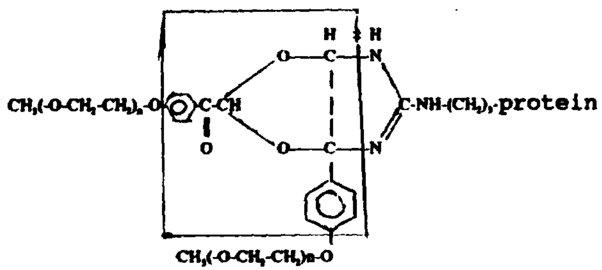 | ‡ secondary amine | | pH8.5,7hr,RT (19) |

| Method | Coupling Moiety (Boxed) and general structure of adduct | Bond | Co-Product | Recommended Coupling Reaction |
|---|---|---|---|---|
| Cyanogen bromide | This process potentially provides three products<br>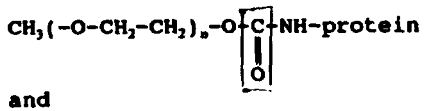 | carbamate | $NH_3$ | 16h,0°C,pH7.5 (20) |
| | and<br>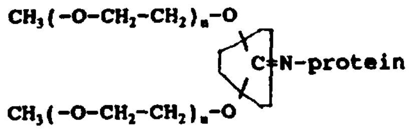 | imido carbonate | $NH_3$ | |
| | and<br>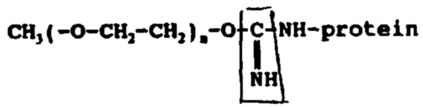 | - | None | |
| Amine acylation | 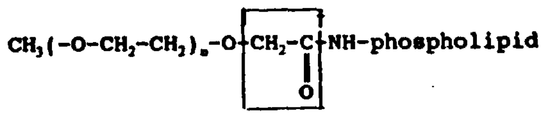 | † amide | PEG-carboxylate | 3h,65°C, benzene (21) |

| Method | Coupling Moiety (Boxed) and general structure of adduct | Bond | Co-Product | Recommended Coupling Reaction |
|---|---|---|---|---|
| PEG-propion-aldehyde | $CH_3(-O-CH_2-CH_2)_n-$ $S-(CH_2)_3-S-(CH_2)_3$ $-NH$-protein<br>or<br>$CH_3(-O-CH_2-CH_2)_n-$ $O-CH_2-CH_2-CH_2$ $-NH$-protein | ‡ secondary amine | oxidised cyano-borohydride | 1H, RT, pH9 (22) |

Notes

1. cf. 2,4,5-trichlorophenol from MPEG 2,4,5-trichlorophenylcarbonate; p-nitrophenol from MPEG-p-nitrophenylcarbonate.
2. Room temperature
3. Abuchowski (1977a)
4. Pyatak <u>et al</u>. (1980) Res, Commun. Chem. Pathol. Pharmacol., 29, 113-127.
5. Yoshimoto, <u>et al</u>. (1986) Jpn. J. Can. Res., 77, 1264-1270.
6. Abuchowski (1984).

**Notes to Table 1 cont'd**

7. Leonard (1984).
8. Katre (1987).
9. Wie et al. (1981) Int. Arch. Allergy Applied Immunol., 64,84
10. Veronese (1985).
11. Beauchamp (1983) and EP-A-0 154 432
12. Pizzo (1991) Advanced Drug Delivery Reviews, 6, 153-166.
13. Zalipsky (1992).
14. Yoshimoto (1987).
15. Aldwin and Nitecki, (1987) Analytical Biochemistry, 164, 494-501.
16. Goodson and Katre (1990).
17. Harris (1989).
18. Delgado (1990).
19. Maeda et al. (1989) EP-A-0340741
20. Iwashita et al. (1981) US-A-4301144
21. Sears (1983) EP-A-0072111
22 Harris et al. New polyethylene glycols for biomedical applications, in Shalaby SW, McCormick CL, Butler GB (eds): Water-soluble Polymers, Washington D.C., American Chemical Society, 1991.

Methods previously disclosed suffer from one or more of the following defects:

1. Substantial loss of biological activity (e.g. 20-95% loss of bio-activity) is frequently seen with the cyanuric chloride and carbonyldiimidazole methods and occasionally with phenylchloroformate and succinimidyl active ester methods.

2. The coupling of PEG (or other polymers) to proteins (or other target molecules) is, with few exceptions, in a manner which leaves part of the activating moiety, the coupling moiety, between the polymer and the target molecule. Of the above methods, only the organic sulphonyl halide methods and a PEG-acetaldehyde method (one of the PEG aldehyde methods [Harris (1989), Harris (1991)]) couple PEG directly without coupling moieties (see Table 1). With the exception of some PEG acetaldehyde methods where the coupling moiety is ethylene oxide (and thus indistinguishable from PEG itself) and the direct coupling methods above, all other coupling methods incorporate a coupling moiety distinct from the polymer and the target and are regarded herein as "indirect" coupling methods.

The incorporation of a coupling moiety generates further problems depending on the nature of the coupling moiety, thus

(i) some coupling moieties provide reactive groups capable of linking further molecules to the polymer-target construct via the coupling moiety (e.g. the triazine ring of the cyanuric chloride method, Leonard, M. et al., Tetrahedron, 40: 1585 (1984));

(ii) some coupling moieties provide an immunogenic/antigenic group (e.g. the triazine ring of the cyanuric chloride method);

(iii) some coupling moieties are potentially toxic or are themselves of unknown toxicity but derived from a compound known to be toxic (e.g. the triazine ring of the cyanuric chloride method and reagents in the phenylchloroformate method); and

(iv) some coupling moieties provide targets for enzymatic cleavage (see below).

3. Coupling in some instances is via an unstable bond liable to be cleaved by enzymes available in serum, plasma, cells or other biological materials or by procedures applied to the polymer-target product. This has two possible deleterious consequences,

(i) the polymer-target construct is degraded enzymatically or by the conditions required for subsequent reaction steps; the former occurs with methods generating amide and/or ester bonds (see Table 1); and

(ii) removal of the polymer alters the target molecule; this occurs with some succinimidyl active ester and mixed anhydride methods,

and either or both of these can occur.

4. Many methods recommend long coupling times and/or unphysiological pH, thus rendering some target molecules less active or inactive (cf. the carbonyl-diimidazole, cyanuric chloride, phenylchloroformate and some succinimidyl active ester methods (see Table 1)).

5. Many methods use activated polymer species and/or produce co-products which are toxic in a wide range of bioassays and which are potentially toxic in vivo if not separated from the product (e.g phenylchloroformate, cyanuric chloride methods).

6. Many methods produce products highly contaminated with either activated or inactivated polymer, causing problems in analytical methods like FPLC and toxicity in some bioassay systems (cf. the organic sulphonyl halide [Delgado (1990)] and phenylchloroformate [Veronese (1985)] methods).

7. Some methods are unsuitable for use in aqueous solution, thus limiting the target molecules to those which will tolerate non-aqueous conditions (cf. the organic sulphonyl halide method using trifluoromethanesulphonyl chloride).

8. Some of the activated polymer constructs are unstable, for instance being subject to hydrolysis during either the activation or coupling reactions (cf. the phenylchloroformate method [Veronese (1985)]). The PEG-acetaldehyde is sensitive to decomposition under basic conditions and can give irreproducible results [Harris et al. (1991)].

The present inventors have previously described PEGylation by an organic sulphonyl halide method using 2,2,2-trifluoroethanesulphonylmonomethoxy PEG (TMPEG) as follows:

1. WO 90/04606 discloses a method for fractionation of PEG-proteins formed by the TMPEG method (discussed below) and an individual adduct, PEG-gm-CSF. The fractionation method is unrelated to the present invention. The PEG-protein products of the processes disclosed in this application, and specifically disclosed materials such as the PEG-gm-CSF adduct, are excluded from the present invention.

2. PCT WO 90/04650 discloses a method to separate DNA/protein complexes using PEG adducts formed by the TMPEG in which PEG acts as an affinity ligand. These complexes are also excluded from the present invention.

3. PCT WO 90/04384 discloses the TMPEG method for attaching PEG to liposomes. Such PEGylated liposomes are excluded from the present invention. It should be noted that the objective of coupling PEG to lipsomes in this patent was to improve half life and liposome stability.

The TMPEG method mentioned above, for instance in WO-A-90/04606, comprises activation of monomethoxy PEG (MPEG) with 2,2,2-trifluoroethanesulphonyl chloride (tresyl chloride) to produce tresyl MPEG (TMPEG) which is subsequently reacted with the target protein molecule to produce monomethoxy products. The same technique is described in WO 90/04650 for coupling monomethoxy PEG moieties to DNA/protein complexes and in WO/04384 for coupling monomethoxy PEG moieties to liposomes. Further investigation of the TMPEG method has revealed that the reactivity of the activated polymer, TMPEG, is severely impaired necessitating the use of uneconomically large ratios of TMPEG to the target molecules and has exposed the rather poor yield of the final products and the low purity thereof, necessitating extensive purification and thereby further reducing the overall yield.

The present inventors have addressed these difficulties in further research and have identified a number of factors which, in combination, contribute to the poor performance of the TMPEG process. This has allowed development of an improved technique for producing PEG adducts. The technique has been extended to include additional technical developments permitting more facile formation of complex adducts of PEG and other polymers and the formulation of adducts of a wider range of target molecules.

The present invention relates to an improved process for producing polymer:target molecule adducts which are directly covalently linked without any intervening coupling moiety residue from the activating group on the polymer and in which the link between polymer and target moieties is non-immunogenic, non-antigenic, non-toxic and non-biodegradable, this objective being achieved by careful control of the properties of certain of the reagents and the reaction conditions. The coupling step may be performed in aqueous media and occurs rapidly thus minimising damage to fragile target species.

Accordingly the invention provides a process for producing an adduct of a polyethylene glycol (PEG) polymer and a target material which process comprises the steps of

a) reacting tresyl-chloride with a polymer of formula (II)

$$(C)_c - PEG - (OH)_g \qquad (II)$$

wherein

the PEG has a valency c+g,
c is zero or a positive number and
g is a postive number

so as to form

a sulphonate ester-activated polymer of formula (III)

$$(C)_c - PEG - (tresyl)_g \qquad (III)$$

wherein

C is a capping group
c and g are as defined above

(b) reacting the activated polymer of formula (III) with the target material and
(c) recovering the adduct of the polymer and the target material,
in which process:

(i) the polymer of formula (II) is dried as obtainable by distillation of any water-benzene azeotrope and then the benzene from a benzene solution of the polymer and said dried polymer of formula (II) is immediately dissolved in an organic solvent which is inert to the reagents and to the product of formula (III) and is anhydrous as obtainable using molecular sieves of 0.3 nm;

(ii) the polymer solution formed in step (i) is reacted with the tresyl chloride in the presence of a base in a reaction vessel from which water is excluded, the polymer of formula III is obtained by removing the organic solvent and dissolving the resultant solid in methanol/hydrochloric acid (1000:0.3), recovering the polymer of formula III as free of base as obtainable by precipitating overnight, recovering the resultant precipitate and supernatant by centrifugation, repeating the dissolving-precipitating-centrifugation steps until no base is detectable in the supernatant;

(iii) the sulphonate ester-activated polymer of formula (III) so produced is recovered dry as obtainable by liquid nitrogen freezing and lyophilisation under reduced pressure;

(iv) the product of step (iii) is then used directly in step (b) or stored as to avoid hydrolysis prior to use in step (b); and

(v) the reaction in step (b) of the sulphonate ester-activated polymer with the target material is conducted in a non-denaturing medium at non-denaturing temperature with respect to the target material.

As used hereinafter reference to the "polymer" is directed towards the PEG.

In further variants of the process of the invention, which can be conducted in conjunction with any of the foregoing variants, the proportion of the tresyl groups on the sulphonate ester-activated polymer which react with the (initial) target material is controlled such that in at least a proportion of the molecules of polymer:target adduct produced there remain unreacted activating tresyl groups Such unreacted reactive groups may then be reacted with one or more different further target materials so as to form materials polymer:[target]$_n$ wherein n is 2 or more and the initial and further target entities are different; or they are reacted with more of the initial target material so as to form materials polymer:[target]$_n$ wherein the target entities are all the same; or they are reacted with further unreacted groups in the initial target entity of the polymer:target adduct (for instance under conditions which hinder intermolecular reactions, such as high dilution) such that the polymer moieties are bonded at two or more positions to the target entity.

It will be appreciated that, since the polymer and/or the target materials may be multivalent, it is possible by the process of the invention to produce a variety of polymer:target structures. By way of example, a univalent polymer and univalent target produce 1:1 adducts; a bivalent target and a univalent polymer may form adducts wherein the target entities bear two polymer moieties whereas a bivalent polymer and a univalent target may produce species where two target entities are linked to a single polymer moiety; use of higher-valent polymers can lead to the formation of clusters of target entities bound to a single polymer moiety whereas higher-valent targets may become encrusted with a plurality of polymer moieties.

In general the target moieties are likely to have more than one reactive group which will react with the activated polymer and the possibility of forming complex structures must always be considered; when it is desired to form simple structures such as 1:1 adducts of polymer and target, or to use bivalent polymers to form target:polymer:target adducts, it will be necessary to use predetermined ratios of activated polymer and target material, predetermined concentrations thereof and to conduct the reaction under predetermined conditions (such as duration, temperature, pH etc.) so as to form a proportion of the described product and then to separate the described product from the other reaction products.

The necessary reaction conditions, proportions and concentrations of the reagents can be obtained by relatively simple trial-and-error experiments with appropriate scaling-up as necessary. It will be appreciated that molar proportions and the concentrations of individual reagents may need to be adjusted on scaling up compared with the optimal molar proportions and concentrations identified at the laboratory bench scale for production of a particular product; this may also require relatively simple trial-and-error experimentation. Purification and separation of the products is similarly achieved by conventional techniques well known to those skilled in the art.

The present invention therefore provides the following particular embodiments of the process:

1. A process as described above comprising, in step (b), reacting, in predetermined molar ratio and at predetermined concentrations, sulphonate ester-activated polymer having two tresyl groups with a first target material, so as to produce a sulphonate ester-activated 1:1 adduct of polymer and target material, then reacting the 1:1 adduct with a second target material so as to produce a target$^1$:polymer:target$^2$ adduct, wherein the first and second target materials may be the same or different, and then recovering the adduct in accordance with step (c).

2. A process as described above comprising, in step (b), reacting, in predetermined molar ratio and at predetermined concentrations, sulphonate ester-activated polymer having only one tresyl group with a target material having more than one reactive group so as to form an adduct of the polymer and target having a preselected number of polymer molecules per molecule of target or, especially for macromolecular targets having a large number of reactive groups, so as to form an adduct in which a preselected proportion of the reactive groups have been reacted with and linked to polymer molecules, and then recovering the adduct in accordance with step (c). The preselected number may be, for instance from 1 to 10, preferably from 1 to 5, for example 1, 2, 3 or 4. The preselected proportion may be, for instance from 1 to 100 %, preferably from 5 to 95 %, for example, 10, 20, 30, 40, 50, 60, 70, 80, or 90%.

3. A process as described above comprising, in step (b), reacting, in predetermined molar ratio and at predetermined concentrations, sulphonate ester-activated polymer having two or more activating tresyl groups with a target material having two or more reactive groups so as to produce a sulphonate ester-activated 1:1 adduct of polymer and target then changing the reaction conditions to hinder intermolecular reactions whilst permitting intramolecular reactions between the terminal carbon atom of the polymer and reactive groups on the target so as to form adducts of polymer and target having two or more covalent bonds between the polymer and target moieties, and then recovering the adduct in accordance with step (c).

These process variants may each be used in conjunction with any of the process variants described earlier relating to liquid phase and solid:liquid phase interface reactions.

The reagents used in the process of the invention will now be described:

Tresyl chloride used in step (a) is the compound:

2,2,2-trifluoroethanesulphonyl chloride

POLYMERS OF FORMULA (II)

The PEG polymers of formula (II) used in the present invention are all based on known and readily available polymers which generally already contain at least one group reactive with the chloride group of the tresyl chloride.

As polymers are almost inevitably mixtures, the value of g will be an average for the material as a whole but is preferably nearly or, more preferably, exactly integral, preferably with a high degree of homogeneity. For polymers which have two or more reactive hydroxyl groups it may be necessary or desirable to prevent reaction at a proportion of such groups by blocking them with a capping group C. The number of capping groups will also be an average for the material as a whole and is preferably nearly or, more preferably, exactly integral, or is zero when no capping groups are required. The PEG polymers, and capping groups C will be further described below.

The PEG element of formula (II) is polyethylene glycol of varying claim length as discussed below

Typically the polymer will be water soluble. The polymer is preferably of very low toxicity in vivo and in vitro, since many applications involve administration to man or animals and/or exposure to cells in cell culture systems. The polymer length is not restricted in this invention, since it will depend on the principal use of the product (e.g. short lengths may be best for coupling two proteins together and longer polymers for improving plasma half life). With each application optimum polymer length must be selected either empirically or by reference to the desired application. The polymer's own inherent hydrophobicity is also important in some settings and will have to be evaluated on a case-by-case basis.

The reactive hydroxyl groups (OH), are terminal hydroxyl groups. Depending upon the selected polymer such groups may already be available for reaction or it may be necessary to introduce such groups by conventional techniques in a preliminary step. Generally the hydroxyl groups are bonded to carbon atoms of the polymer. The hydroxyl groups must be capable of reacting with the chloride group of the tresyl chloride. The hydroxyl groups are such that they ultimately permit formation of the desired non-immunogenic, non-antigenic, non-toxic, non-biodegradable direct covalent link between the polymer and target. Such hydroxyls react with tresyl chloride to form activating sulphonate ester moities.

The use of capping groups C is an important feature of the reaction since this embodiment of the invention has special advantages discussed further below. The function of the capping groups C is to protect one or more reactive hydroxy groups of the polymer molecule from nucleophilic attack while leaving the activated polymer product susceptible to nucleophilic attack only at the carbon or equivalent atom adjacent to the tresyl groups. C therefore must lack the properties of tresyl and should preferably be an inert group with respect to reactivity with other molecules and should be of low toxicity. Suitable groups C are well known to those skilled in the art as are techniques for introducing such groups. A preferred group C is methyl.

Production of the polymers for use in the process of the invention may be achieved by conventional techniques although most materials will be obtained commercially, possibly then being modified to introduce the desired hydroxy reactive groups and/or capping groups C as mentioned above.

The preferred polymer for use in accordance with the present invention is a PEG polymer HO-$(CH_2CH_20)_x$-H where x is large enough for the molecular weight to be in 150(x=3), 194(x=4), 238(x=5), 282(x=6) or above, for instance about 500, 1000, 2000, 3000, 4000, 5000, 6000 or 10000 which has two reactive hydroxyl groups. The size of the polymer actually used will be selected according to the desired properties of the end product.

Another preferred polymer is MPEG (i.e. the polymer MeO-$(CH_2CH_20)_x$-H which has one capping group C, i.e. the methyl group and one reactive group G, i.e. the hydroxyl group) where x is 3, 4, 5 or 6 or is large enough to provide molecular weights of, for instance, about 500, 1000, 2000, 3000, 4000, 5000, 6000 or 10000. Branched PEGs can also be used.

Since conventional polymerisation techniques generally produce materials which are mixtures, in certain cases some purification may be required before the polymer is used in the process of the invention, for example, some preparations of MPEG are significantly contaminated with PEG. This would lead to the production of biactivated PEG and crosslinking on subsequent use and needs to be avoided for many applications. Such contaminated material can be identified on the basis of its size distribution (polymerisation occurs at both ends of the molecule for PEG hence the contaminant has circa twice the molecular weight). Size fractionation should therefore be used to remove this PEG from MPEG preparations prior to activation: gel permeation chromatography and other appropriate methods may be used. For example, the divalent PEG can be separated from the bulk MPEG using vesicle chromatography [Selisko et al (1993) J. Chromatogr. **641**, 71-79].

TARGET MATERIALS

Suitable target materials to which polymer can be attached in accordance with the present invention are all materials having biological activity which are useful in, for instance diagnosis or therapy and which are all well-known to those skilled in the art. They all contain at least one reactive group (hereinafter referred to as groups "N") containing an atom which is capable of mounting a nucleophilic attack on the carbon or equivalent atom of the polymer adjacent to the group AM. Examples of the reactive group include primary and secondary amino groups, thiol groups and aromatic hydroxy groups.

More specifically, potential targets include proteins, peptides, amino acids and their derivatives such as: antibodies and fragments thereof; cytokines and derivatives or fragments thereof, for example, the interleukins (IL) and especially the IL-1, IL-2, IL-3, IL-4, IL-5, IL-6, IL-7, IL-8, IL-9, IL-10 and IL-11 subtypes thereof; colony stimulating factors, for example granulocyte-macrophage colony stimulating factor, granulocyte-colony stimulating factor (alpha and beta forms) and macrophage-colony stimulating factor (also known as CSF-1); haemopoietins, for example erythropoietin, haemopoietin-alpha and kit-ligand (also known as stem cell factor or Steel factor); interferons (IFNS), for example IFNalpha, IFNbeta and IFNgamma; growth factors and bifunctional growth modulators, for example epidermal growth factor, platelet derived growth factor, transforming growth factor (alpha and beta forms), amphiregulin, somatomedin-C, bone growth factor, fibroblast growth factors, insulin-like growth factors, heparin binding growth factors and tumour growth factors; differentiation factors and the like, for example macrophage differentiating factor, differentiation inducing factor (DIF) and leukaemia inhibitory factor; activating factors, for example platelet activating factor and macrophage activation factor; coagulation factors such as fibrinolytic/anticoagulant agents including heparin and proteases and their pro-factors, for example clotting factors VII, VIII, IX, X, XI and XII, antithrombin III, protein C, protein S, streptokinase, urokinase, prourokinase, tissue plasminogen activator, fibrinogen and hirudin; peptide hormones, for example insulin and calcitonin; enzymes such as superoxide dismutase, glucocerebrosidase, asparaginase and adenosine deaminase; vaccines, for example hepatitis-B vaccine, malaria vaccine, melanoma vaccine and HIV-1 vaccine; transcription factors and transcriptional modulators; carbohydrates, glycosoaminoglycans, glycoproteins and polysaccharides; lipids, for example phosphatidyl-ethanolamine, phosphatidylserine and derivatives thereof; sphingosine; steroids such as cholesterol and derivatives thereof; nucleotides, nucleosides, heterocyclic bases, DNA, RNA, synthetic and non-synthetic oligonucleotides including those with nuclease resistant backbones; vitamins; antibiotics; bacteristatic and bactericidal agents; antifungal, anthelminthic and other agents effective against infective agents including unicellular pathogens; small effector molecules such as noradrenalin, alpha adrenergic receptor ligands, dopamine receptor ligands, histamine receptor ligands, GABA/benzodiazepine receptor ligands, serotonin receptor ligands, leukotrienes and triiodothyronine; cytotoxic agents such as doxorubicin, methotrexate and derivatives thereof.

The target molecules may also be part of larger multi-molecular structures. These include cells or parts thereof, for instance erythrocytes, erythrocyte "ghosts" and leukocytes, liposomes such as multilamellar vesicles and unilamellar vesicles, micelles and micelle-like structures, and aggregates, microemulsions, coacervates, emulsions, suspensions of the foregoing.

It will be appreciated that when the target molecules are part of such structures there will generally be many target molecules in each structure; treatment according to the invention will therefore produce a structure bearing many polymer moieties. Where the polymers are bi- or multivalent, reaction with a multimolecular target may result in intermolecular cross-linking by the polymer between molecules of the same target structure and/or between molecules of different target structures as well as intramolecular bonding of the polymer to more than one position on the same molecule of a target structure.

It is evident from the inventors' investigation that much of the loss of biological activity frequently observed with prior polymer coupling methods is due to inappropriate coupling reactions (i.e. reactions taking place at sites which disable the target's biological activity), coupling conditions and/or contaminating toxic material reducing responses in bioassays. The present process provides a means of generating adducts of polymer and target with highly conserved biological activity for the majority of molecules, or even enhanced biological activity. In the event of unusually substantial loss of biological activity, a search must be made using fractionated material with different degrees of substitution and appropriate mapping (e.g. peptide mapping) to identify the group or groups N the modification of which by polymer is responsible for lost activity. The situation can then be remedied either by modifying the group N to abrogate coupling of polymer at that site or by shielding N with an appropriate non-covalent bond (examples include hydrogen bonding of base pairs for DNA and protein/protein binding like receptor ligand interactions). Two alternative strategies are available depending on the nature of the critical N group, in particular whether there are significant differences in the susceptibility of the group compared to the other such groups (either being more or less susceptible) to coupling to the polymer. If the group N is less susceptible the coupling conditions (usually the coupling ratio) can be altered so that the critical group N is rarely modified. If it is more susceptible, a two step procedure where short polymer molecules are first linked to more accessible (susceptible) sites (i.e. including the group(s) N) then long polymer molecules are linked to the remaining sites may prove acceptable, the outcome depending on the nature of the problem at the N site with respect to biological activity.

Targets lacking a reactive group may be modified so as to create one or more reactive groups; this is within the ability of those skilled in the art and can be achieved by well-known techniques.

Some target structures (e.g. RNA and single stranded DNA) pose special problems because they provide too many "N" groups to which the polymer would attach in a standard reaction. There the groups N may be temporarily protected by involvement in an appropriate conformation precluding the nucleophilic attack, as for example in the hydrogen bonding associated with base pairing of DNA (see below).

An embodiment of the present process involves site-specific modification of DNA, RNA and synthetic oligonucleotide targets (or of any molecule containing an amino or other reactive group which can participate in interactions such as hydrogen bonding with another molecule or compound) by precluding the nucleophilic attack on the activated polymer species by reactive groups on the target. The bases adenine (A), cytosine (C) and guanine (G) [but not uracil (U) or thymine (T)] provide suitable targets in DNA, RNA and synthetic oligonucleotides for modification with polymer moieties according to the invention and thus these are special targets with the problem that there are too many available reactive groups to which the polymer can be attached. As shown below, single stranded DNA is rapidly and very heavily modified by activated PEG (TMPEG).

By using various restriction fragment DNA cleavage sites as a model system, the present inventors have shown that selected bases A, C or G can be modified by the expedient of leaving short stretches (e.g. 2-4 bases) single stranded. Adenine bases appear to be the most susceptible to such modification. Blunt ended double stranded DNA is not readily coupled under the conditions described, indicating that hydrogen bonding between base pairs is sufficient to preclude interaction of the amino groups of A, C and G bases with the activated polymers.

Site-specific DNA modification by polymer can be achieved by the expedient of including one or more A, C or G bases in a short single stranded section of DNA by appropriate restriction enzyme digestion or by hybridising oligonucleotides of dissimilar lengths with the DNA to protect bases which are not to be modified or by exploiting the natural strand asymmetry of polymerase chain reaction products which have a one base-pair overhang, or by exploiting localised regions of single strandedness achieved by natural or artificial localised melting of the double helix.

<u>THE PROCESS</u>

The process of the present invention relies upon conducting steps (a) and (b) in accordance with certain constraints as defined above. In particular step (a) must be conducted using steps (i) to (iii) as described above. Thus, dryness of the polymer may be determined by benzene distillation as described below. If the polymer is not sufficiently dry, interfering side reactions occur making it difficult to obtain economical amounts of the final adduct. Likewise, wet solvents and ingress of water during the reaction will also cause interfering side reactions. Suitably dry solvents are obtained using molecular sieves (0.3nm) as described below. Exclusion of water from the reaction vessel can be achieved by conventional techniques.

<u>Benzene distillation</u>

The principal exploited here is that the water-benzene azeotrope has a boiling point of 65°C and that after water has been removed from a sample, the distilling temperature rises to that of pure benzene (i.e 79 to 80°C). In addition, the water-benzene azeotrope is cloudy and benzene is clear so that the change from cloudy to clear distillate confirms that drying is complete. If a suitably dry sample is heated in benzene the distillation temperature will be that of pure benzene and the distillate will be clear from the first.

<u>0.3 nm Molecular sieves</u>

Solvents may be dried by any conventional method provided that they are dried to the extent obtainable by use of 0.3 nm molecular sieves. Thus, for instance, when 0.3 nm molecular sieves are used an amount of molecular sieve is added to and left in contact with the solvent for several hours (preferably about 10 hours or more) and at about room temperature (ca 20°C), the amount of molecular sieves used suitably represents a twenty fold excess over the recommended amount to adsorb the anticipated water content in the solvent, according to the manufacturer's instructions, for instance about 100g of molecular sieve per litre of substantially dry solvent.

It is also a requirement that the solvent should be inert to the reagents and the activated polymer. Suitable inert solvents include halogenated alkanes such as dichloromethane and other organic solvents with low hydrophilicity and low hygroscopicity; such solvents are well known to those skilled in the art. Halogenated alkanes, especially dichloromethane, are preferred.

The activated polymer is preferably used directly following its production in accordance with step (a) as this affords the minimum opportunity for degradation leading to formation of other reactive species which will interfere with subsequent reactions. If the product is to be stored prior to use, for instance when formed in bulk for use in smaller quantities at a later date, it must be stored so as to avoid hydrolysis, i.e. in the presence of a more hygroscopic desiccant. Sulphonyl halides are highly hygroscopic and will dry many common desiccants such as silica gel and thus become hydrolysed. The preferred desiccant for drying and storage of the activated polymer is phosphorus pentoxide.

The reaction of the activated polymer with the target material in step (b) takes place in a non-denaturing medium; for many biological materials this will of necessity be an aqueous medium. There are also many target materials which are stable under non-aqueous conditions and for these it is quite acceptable to conduct step (b) in organic solvents which are inert to the reagents. Ultimately the choice of reaction medium will be dependant on the stability of the target material and final adduct in that medium. Selection of pH, salt concentration, protein concentration and other requirements for stability will be determined on a case-by-case basis. Those skilled in the art will have no difficulty with this. The same applies in relation to determination of non-denaturing temperatures.

When the process of the invention involves the use of a solid support coupled to the activated polymer, the reactions involved in coupling the activating moiety to the solid support will also be conducted under dry conditions, i.e. dry reagents as determined by benzene distillation and dry solvents as determined using molecular sieves of 0.3 nm, will be employed whenever sulphonyl halide groups are present.

It is preferred that the reaction of step (a) is conducted in the presence of a base such as a tertiary amine, preferred bases include those which are easily removed by washing, such as pyridine, and volatile bases which may be removed by evaporation. At present the preferred base is pyridine. However certain bases, especially pyridine, may react deleteriously with The target material and these should be removed prior to step (b). This may be achieved by conventional means.

It is preferred That the reagents for step (a) are mixed at reduced temperature, preferably at 0°C for instance by chilling with ice and that The reaction mixture is then allowed to wan to ambient temperature, for instance about 20°C.

These preferred features may be adopted in any combination but most preferably all of these preferred features are adopted.

When the polymer of formula (II) has more than one hydroxy group and/or one or more capping groups C, it has been found that contamination with related products of formula (II')

$$(C)_{c'}\text{-PEG-}(OH)_{g'} \qquad (II')$$

wherein C, is as defined above and c' and g' are zero or positive numbers such that $c'+g' = c+g$ but g' is different to g, can lead to undesirable inhibition of the intended reaction and formation of unwanted by-products which therefore complicates the purification and recovery of the eventual adduct. In the case where the polymer of formula (III) is a polyalkylene oxide with one capping group (eg MPEG), c is 1 and g is 1. Contaminants of formula (II') in which c' is 2 and g' is 0 will be unreactive and thus not detrimental but contaminants in which c' is 0 and g' is 2 will cause cross-linking of target materials; preferably such contaminants are avoided in production of the compounds of formula (II) or are removed prior to use of the compound in step (a) of the present process. When the polymer of formula (II) is a material in which $c' + g' > 2$ there are many possible contaminants; again those wherein g' is 0 are unreactive and of little concern whereas those wherein $g' > 1$ and $g' > g$, especially those wherein $g' > g$, cause unwanted side reactions and are preferably removed from the reagent before use.

Whilst the reaction of step (b) will proceed in the presence of large amounts of such contaminants, it is preferred that the amount of all compounds of formula (II') present in the compound of formula (II) is less than 35%, for instance less than 25%, more preferably less than 20%, for instance, 15, 10 or 5% and yet more preferably below 1% by weight based on the total weight of the compound of formula (II) and all compounds of formula (II'). This may be achieved by purifying the starting materials used in forming the polymer, controlling the polymerisation and subsequent process steps required to form the compound of formula (II) and/or by purifying the compound of formula (II) before use in the process of the invention.

In a preferred aspect of the invention the compound of formula (II) is MPEG in which PEG is a divalent residue $-0-(CH_2CH_20)_x-$ (for instance the residue of PEG 5000), c is 1 and C is methyl and g is 1. This material is produced by polymerisation from methoxyethanol and ethylene glycol and tends to contain a large proportion of material where polymerisation has initiated from ethylene glycol rather than the methoxyethanol such that chain extension occurs in both directions and a PEG product of approximately twice the molecular weight of the MPEG is present as a major contaminant; this may readily be removed by gel permeation chromatography at an appropriate molecular weight cut-off.

Purity can be checked by nmr and chromatography. Contamination by materials where g' > g is particularly to be avoided when 1:1 target:polymer adducts are to be produced, since the presence of such contaminants will lead to the production of aggregated materials.

When the reaction of target and activated polymer is to be performed at a solid:liquid interface, the activated polymer may, in one variant of the process, be linked to a solid support after reacting a compound of formula (I) having a suitable linking group with the polymer. The linking reaction may be effected as described above in connection with the production of compounds of formula (I) linked to a solid support. Thus, for instance, a tetrafluoro-4-amino-azobenzene-4'-sulphonyl derivative of the polymer is reacted with a solid support bearing aldehyde groups (for instance siliconised glass treated with glutaraldehyde) in the presence of a non-nucleophilic base, such as lithium isopropylamide, followed by borohydride reduction of the potentially labile double bond.

It is particularly preferred that the process of the invention is conducted using tresyl chloride and MPEG, for instance MPEG 5000, as the polymer of formula (II). For MPEG of molecular weight up to 5000 or 6000, purity can be checked by nmr but for higher molecular weight material the methyl signal is swamped and chromatographic techniques must be adopted. These are well known to those skilled in the art.

In this preferred aspect, the product of formula (III) is TMPEG. Ideally this is used immediately but, if not, storage of TMPEG is critical and activity is not well maintained unless it is properly stored. When desiccated over phosphorus pentoxide and stored at 4°C, activity is maintained for at least 1 year. Degradation of TMPEG (either during production or on storage) is detectable on the basis of a change in the FPLC profiles of exposed peptide targets (see Example 1). It produces less substitution of the target at a given molar ratio (a loss of resolution and right shift of the elution profile accompanies the former if FPLC loads are not corrected for the amount of PEG loaded). Substantial aging of TMPEG prepared as previously described is documented in Figure 8 in WO90/04606. Since degradation produces coproduct, degraded TMPEG preparations are also more acidic.

PEG itself is known to oxidise on storage to a brown discoloured product. The TMPEG preparation process removes the antioxidants present in commercially supplied PEG. Oxidation has been observed in several stored TMPEG preparations and it is recommended that, if prolonged storage is anticipated, an appropriate antioxidant should be included. This new method for preparing and storing the TMPEG has significant advantages over previously described methods in terms of the activity, reproducibility between preparations and stability of individual preparations.

In an especially preferred aspect the present invention provides a process comprising the steps of

(a) reacting dry MPEG (as determined by benzene distillation) containing less than 10% by weight of PEG (as determined by nmr and/or chromatography) with tresyl chloride in dry dichloromethane (as determined using 0.3 nm molecular sieves) in the presence of pyridine in a reaction vessel from which water is excluded, so as to form TMPEG, recovering the TMPEG so formed and either storing it over phosphorus pentoxide or using it directly in step (b),

(b) reacting the TMPEG so formed, optionally after storage, with a target material such as erythropoietin (EPO) in aqueous medium and about 20°C and

(c) recovering the Target:MPEG adduct (eg EPO:MPEG) so formed.

More preferably the molar ratio of tresyl chloride to MPEG is 2:1 or more, most preferably about 2.5:1.

The process of the present invention may be supplemented by one or more separation and/or purification steps at any stage as necessary or desirable. Separation and purification may be achieved by appropriate techniques well known in the art selected having regard to the need to maintain the activity of the reagents and products and to avoid use of toxic or interfering materials. In some cases, for instance where the polymer is a polyalkylene glycol, aqueous biphasic polyalkylene glycol solutions may be used to effect separation and/or purification of the activated polymer and/or polymer:target adduct.

THE PRODUCTS

The present invention further provides polymer:target adducts obtainable by the process of the invention other than the known polymer:target adducts having polymer and target moieties directly linked by stable, non biodegradable covalent bonds which are described in the references discussed above.

The invention also extends to such products for use in therapeutic and diagnostic methods of treatment of the human or animal body and to the use of such products in the manufacture of medicaments for use in therapeutic and diagnostic methods of treatment of the human or animal body and to pharmaceutical compositions comprising products of the invention together with pharmaceutically acceptable diluents or carriers.

Many of the prior methods of coupling polymers to targets are unsuitable for coupling more than one target molecule to each polymer molecule since most methods use polymers having only one group capable of reacting with the target. In contrast, the process of the present invention enables the production of constructs such as the following which form particular embodiments of the present invention:

1. Liposomes bearing linear polymeric linking groups bound to the surface of the liposome at one terminal of the polymer and bearing a receptor ligand, receptor molecule, antibody, antigen or other molecule of therapeutic or diagnostic interest at the other terminal.

2. Constructs where a plurality of identical target molecules are linked to a single polymer moiety; for instance a linear polymer having two reactive termini is linked to two target molecules to enhance the biological activity thereof or a branched polymer having three or more reactive termini or a polymer having reactive pendant groups is linked to three or more target molecules.

3. Constructs as in 2. above wherein the target molecules are different; such constructs may be intended to have synergistic effect when the target molecules interact with each other or with other substrates.

4. Constructs wherein the polymer moiety is linked to a target molecule at two or more positions either by reaction of both termini of a linear polymer with different sites on the target molecule or by reaction of pendant reactive groups, or the termini of a branched polymer, at two, three or more sites on the target molecule.

The products of the present invention preferably comprise any one of the polymer materials set out above and any one or more of the target materials set out above. Particularly preferred products of the invention are adducts of a polyalkylene glycol, especially polyethylene glycol and polypropylene glycol with the former being most preferred, with any one or more of the target materials set out above, with erythropoietin (EPO) being particularly preferred.

The production of adducts wherein two or more different target species are coupled to a multivalent polymer presents special difficulties which can be overcome by the process of the present invention. For this situation an appropriate procedure for producing a product comprising two different targets involves the following steps:

1. A first target, Target (A), is exposed to activated polymer (tresyl-PEG-tresyl) with the latter in gross excess such that essentially only 1:1 Target(A):PEG adducts are formed and 2:1 or higher Target(A):PEG adduct formation is minimised. This can be followed by FPLC or other appropriate methods to separate and, if necessary, purify the adduct, provided that this is sufficiently rapid to avoid hydrolysis of the remaining activating moieties. If the target has many derivatisable groups, it may be necessary to fine-tune the coupling ratio, reaction time and/or pH, so that 2:1 or lower Target(A):PEG adducts are not formed or, if formed are discarded. Alternatively a mixture of monovalent and multivalent activated polymer species may be used in a ratio selected such that it is statistically likely that only one of the polymer molecules attached to any target molecule will be the multivalent form (targets which are by chance only modified with the univalent form will be wasted).

2. Either:

a) the Target(A):PEG adduct is first separated from the tresyl-PEG-tresyl then reacted with the second target, Target(B), preferably at equimolar ratio or, if this reacts too slowly, with the adduct in excess; or

b) the reaction product of step 1 is reacted with Target(B), preferably at a molar ratio of 1:2 with respect to the amount of activating moieties remaining in order to reduce the formation of Target(B):polymer:Target(B) constructs. This is much simpler than (a) but more wasteful in terms of Target(B) which is therefore selected as the cheaper of the targets.

After a sufficient reaction time (derived empirically), the excess tresyl is neutralised with a monovalent neutralising agent e.g. with glycine, and the products are purified to discriminate the combinatorial possibilities. This may be possible on the basis of molecular weight if target(A) and target(B) differ significantly in size or via differential labelling of the targets. Alternately, since the major contaminant will be Target(B):polymer:Target(B) at this stage, an affinity purification method, with affinity ligand to Target(A), should enrich for hybrids. Similar measures may be adopted to construct more complex adducts.

A particular advantage of the present invention is that the process enables the coupling of polymer moieties to oligonucleotides and nucleic acids (DNA and RNA) at defined positions. EP-A-O 292 128 (Seger 1988) deals with "improved DNA probes" and uses PEG, amongst other molecules, to link DNA to reporter groups for hybridisation.

However the PEG is linked to the 5'-phosphate of the DNA and there is no indication of a method which would link the PEG without leaving a coupling moiety. This ability to modify DNA, RNA and synthetic oligonucleotides with polymer will influence the solubility and alter biodistribution and has applications in both the in vitro and therapeutic or diagnostic (in vivo) use of nucleotides.

Further improvements offered by the invention include the ability to maximise the biological activity retention and/or to increase the activity of the target molecule, minimise the toxicity of the product, minimise the reaction time at physiological pH, reduce contamination of the product and improve the stability of the activated polymer.

The invention will be illustrated with reference to the accompanying figures of the drawings in which:

Fig.1 shows the results of separations of MPEG 5000 obtained commercially.

Fig.2 is a plot of age of TMPEG used and % of gm-CSF left unmodified by reaction with aged TMPEG produced according to the invention.

Fig.3 is a plot of the relative activities of PEGylated gm-CSF prepared by the process of the invention and by a previously described process.

Fig.4 plots the activity of EPO and PEG-EPO.

Fig.5 is a gel showing DNA fragments variously treated with TMPEG, MPEG or buffer.

Fig.6 shows log concentrations of qm-CSF and various PEG-gmCSF adducts in blood and tissue of mice.

Fig.7 shows the results of fractionation of gm-CSF and PEG-gm-CSF before and after ageing.

Fig.8 shows FPLC profiles for EPO PEGylated with various molar ratios of TMPEG:lysine.

Fig.9 shows biological activity of unmodified and PEGylated EPO.

The present invention will now be illustrated by the following Examples which are not intended to limit the scope of the invention in any way:

## EXAMPLES

### Examples 1a and b and Comparative Examples 1a and b

(a) Purification of MPEG

MPEG-5000 (Union Carbide) was subjected to permeation gel chromatography on Superose 12 (Fig. 1a). The material has a clearly biphasic size distribution with an estimated 22.9% high molecular weight contaminant (9280Da) and a low molecular weight component (5460Da). The good agreement between proportions of the two peak areas and the NMR estimate of PEG versus MPEG in the same preparation suggest that all the PEG is accounted for by the high molecular weight species. To confirm this and to devise an appropriate purification procedure, vesicle chromatography [Ehwald (1991)] was used. Analytical and preparative scale preparations gave different efficiencies of separation. Analytical scale separation was performed using vesicular packing material P1(5) equilibrated in 0.05M $NaH_2PO_4$ buffer pH 5.5 (column bed volume 22 ml). The MPEG preparation (Union Carbide Mw 5000), 0.5ml of a 20mg/ml solution in PBS was loaded onto the column. The column was eluted with PBS using a flow rate of 0.08 ml/min and 0.5 ml fractions were collected. The elution profile is shown in Fig.1b; circa 8% of the material was eluted in the excluded fraction (high molecular weight) and 92% in the permeable fraction (low molecular weight fraction). In preparative scale (using 6.5L bed volume and 15g/500ml MPEG in 0.01M $NaH_2PO_4$ pH 5.5 and an elution rate of 20.5 ml/min) the excluded fraction (PEG) was 11.2 % (Fig.1c). Rechromatography on Superose 12 of the two pooled fractions (shaded and hatched areas of Fig.1c) taken from the preparative scale fractionation on Superose 12 was used to determine the extent to which the two materials were separated (Fig. 1d, squares - permeable fraction, crosses - excluded fraction). NMR analysis of the permeable fraction gave an estimated ratio of PEG:MPEG of 11:89 mole:mole, which is in good agreement with the area under the curve estimate of the residual high molecular weight contaminant in the permeable fraction on the elution profile on Superose 12 (Fig.1d, squares). NMR analysis could not be performed on the excluded fraction because of its much higher molecular weight. Further rounds of vesicle chromatography, at analytical scale, gave a comparable reduction in the high molecular weight material (the permeable fraction yielded a smaller "excluded" peak on rechromatography, circa 4% in the high molecular weight excluded fraction, using a second round of vesicle chromatography, Fig. 1e).

(b) Preparation of TMPEG

Tresyl chloride was used when freshly prepared or after storage in airtight ampoules. MPEG-5000 (Mr 5000, 18g, 3.5 mmol) was dried with benzene (B.P. 79-80°C) by distillation of the water-benzene azeotrope and then the benzene. The water-free MPEG was then immediately dissolved in dichloromethane (45 ml, ANALAR, previously dried over molecular sieve 3Å, 100 g per litre of solvent, overnight at room temperature). Activation of MPEG-5000 with tresyl chloride was carried out at a molar ratio of 2.5:1 (tresyl chloride to available hydroxyl groups in MPEG). The MPEG/dichloromethane mixture was then cooled on ice (to circa 3°C) and pyridine (1 ml, 12.4 mmol) followed by tresyl chloride (1 ml, 9 mmol), both precooled on ice were added dropwise with constant stirring using a magnetic stirrer. The reaction was allowed to continue (for 2 hr) at room temperature with constant stirring before the removal of dichloromethane by evaporation under reduced pressure. The solid obtained (TMPEG) was redissolved in methanol-hydrochloric acid mixture (1000:0.3 v/v) and allowed to precipitate overnight at reduced temperature (-20°C). The precipitate was recovered by centrifugation (at 1100 x g, for 10 minutes) at reduced temperature (0°C) and the supernatant was spectrophotometrically checked for pyridine content (λmax 255nm). This procedure was repeated until no pyridine could be detected, usually 12 to 15 washes. Finally, the pyridine-free precipitate was washed in methanol only, preferably at least twice, and then the methanol was removed by evaporation under reduced pressure and the TMPEG was finally dried by freezing in liquid nitrogen and lyophilising under reduced pressure. The hydroxyl content of the white solid as determined by $^1$H nmr obtained was undetectable (4.56ppm). Therefore, approximately 100 % of hydroxyl groups in the MPEG were transformed into tresyl esters.

The TMPEG so produced was desiccated over phosphorus pentoxide and stored at 4°C.

(c) Coupling to GM-CSF

The activity of TMPEG samples was monitored by exposure of the samples to gm-CSF at molar ratios of 305:1 (Example 1a and Comparative Example 1a) or 75:1 (Example 1b and Comparative Example 1b) in a standard coupling reaction and estimating the percent of the preparation remaining unmodified after the reaction as shown in Fig.2: the relative activities of TMPEG preparations obtained as described above (Examples 1a and b) and of TMPEG made by a prior art process (Comparative Examples 1a and b) is plotted against the storage period. TMPEG made by the process of the invention and used at 305:1 (squares), throughout the observation period, gave <10% residual unmodified gm-CSF. The equivalent result when using the same material at 75:1 (diamonds) was circa 20% unmodified material. With both preparations there was no evidence of a trend of worsening activity with storage. In contrast preparations made from MPEG as obtained from the manufacturer by a previously described method (WO90/04606, Example 1) (triangles; downwards triangles 305:1 and upwards triangles 75:1) showed: (1) lower mean activity (reflected in higher mean residual unmodified material); (2) a very broad scatter of reactivities; (3) 3 samples completely inactive at 75:1 and 2 inactive at 305:1 after only 5-12 weeks of storage. At 305:1 the process of the invention consistently produced 92-95% modification, irrespective of the age of the TMPEG and using a coupling ratio of 75:1, circa 80% of the gm-CSF preparation was modified.

The process described above (Example 1) has been repeated on a number of occasions with similar results.

The gm-CSF PEGylated using the process of Example 1 showed no significant loss of its growth stimulating activity as assessed by granulocyte-macrophage colony assay (Fig.3a) or by thymidine uptake (Fig. 3b). Dose response analyses are a relatively insensitive means for demonstrating subtle differences in bioactivity and are also difficult to analyse after pooling because of the high contribution to errors of the variation in the response level between experiments. A better approach is to perform a correlation analysis, plotting the response obtained with equivalent amounts of the modified and unmodified materials on an XY plot. Identical activity gives datum points randomly distributed about the line representing equal activity (the dashed lines). Reduced activity reveals itself as a curvilinear departure from the equal activity line which converges towards the line at the extremities (if sufficiently broad a dose response range is examined such that the plateau of maximum stimulation is reached). In contrast, the presence of inhibitory material, with or without loss of intrinsic activity, produces a departure, usually without convergence to the upper extremity. In the latter case (i.e. where there is no loss of intrinsic activity) failure to observe divergence at the lower extremity indicates that the inhibitor is masking activity only at relatively high inhibitor levels; divergence throughout the plot indicates either an inhibitor with powerful effect at low dilution or combined inhibition and loss of activity.

Two assays for the growth stimulating activity of GM-CSF prepared by the methods of Example 1 in Fig.3) and Comparative Example 1 (□ in Fig.3) show that the process of the invention gives better conservation of biological activity. Colony counts or 3H-thymidine uptake (normalised with respect to the maximum per experiment to avoid a contribution of between-experiment differences to the correlation) were plotted for comparable amounts of PEG-GM-CSF and GM-CSF over the dose range spanning the upper and lower asymptotes of the dose response curve (0.1 to 8 ng/ml for colony assays and 0.0006 to 1.0ng/ml for thymidine uptake assays). Datum points are means of colony counts in triplicate dishes for three independent experiments (Fig. 3a) or of two independent thymidine uptake experiments (Fig 3b). The results in the thymidine uptake demonstrate no significant loss of activity with the process of Example 1 and loss of activity without significant inhibitory material for the process of Comparative Example 1 (TMPEG in equivalent doses was not inhibitory in this assay). The results for colony stimulating activity again show no significant loss of activity with process of the invention but indicate an additional inhibitory activity for this assay when the old method was used possibly accompanied by loss of bioactivity.

Example 2

a) Preparation of ditresyl PEG (TPEGT)

PEG-6000 (10.5 g, 1.75 mmol) was dissolved in benzene (30 ml) and dried as in Example 1 above. The dry PEG-6000 was then immediately dissolved in 25 ml of dichloromethane (dried as in Example 1), cooled on ice and pyridine (1 ml, 2.4 mmol) followed by tresyl chloride (1 ml, 9 mmol) were added dropwise. The mixture was left to react (two hours at room temperature) and the dichloromethane was evaporated as in Example 1. The solid was resuspended in methanol:hydrochloric acid and was treated as above.

b) Coupling to GM-CSF

To test that this material was bivalent and could couple target(s) at both ends of the PEG chain, this TPEGT was used to aggregate GM-CSF. $^{125}$I-GM-CSF (1.4nmol/ml) was reacted with TPEGT (160mg/ml) for 2h at room temperature. The resulting aggregates were estimated by FPLC and contained 95% of the total GM-CSF. Thus the bivalent AM-polymer-AM construct is demonstrated to be functional.

Example 3

Example 2 was repeated using TMPEG prepared from a sample of MPEG containing 8% PEG as contaminant, and thus containing about 8% TPEGT. When used to aggregate GM-CSF, this material (160 mg/ml) produced an aggregate estimated by FPLC to contain only 20% of the total GM-CSF. This demonstrates that the higher proportion of conjugates seen in Example 2 was related to the additional TPEGT and not merely due to non-specific aggregation. It should be noted that using a prior art process, the present inventors have shown [Malik et al (1992) Experimental Hematology, **20**, 1028, fig. 4] that both the non-specific aggregated gm-CSF in unPEGylated reactions and aggregates generated in PEGylation reactions (which include cross-linked products due to TPEGT) have an extremely high specific activity, as assessed by thymidine incorporation, with respect to monomeric gm-CSF). Thus it is anticipated that the bivalent 2-polymer construct generated in Examples 2 and 3 would have a higher specific activity than monomeric unmodified gm-CSF.

Example 4 and Comparative Examples 2,3 and 4

To document the better conservation of biological activity, direct comparisons were made with three of the widely used current methods, using erythropoietin as the target peptide. It is an important prerequisite in experiments of this type to perform preliminary experiments (using at least two independent assays of bioactivity and some measure of the degree of modification) to select the optimum coupling ratio and duration of coupling reaction for maximum retention of biological activity. It is important to record the degree of modification since this may vary between the methods being analysed and only materials substituted to approximately the same extent should be compared for retention of activity.

Serial dilutions of TMPEG produced as in Example 1 were mixed with the same amount of erythropoietin and the resultant PEG-erythropoietin adducts were assessed first by FPLC for the degree of modification and also in the thymidine uptake assay [Malik et al., (1992) Experimental Hematology, 20: 1028] for a preliminary estimate of activity. Based on the latter a smaller range of activities were analysed in colony assays using the methyl cellulose assay system of Ash et al., Blood, 58:309-316 (1981), Knusli, C. et al. (1992) Br. J. Haematol. 82(4): 654.

Commercially available activated PEGs (Sigma Limited) were used to PEGylate recombinant human erythropoietin (Cilag Ltd.) by the cyanuric chloride (Comparative Example 2), succinimidyl succinate (Comparative Example 3) and phenylchloroformate (MPEG-p-nitrophenylcarbonate) methods (Comparative Example 4). TMPEG, prepared as described above, or the alternate activated PEGs, were incubated (for 2 h) with erythropoietin (100 µg/mli total protein, i.e. carrier plus erythropoietin) in coupling buffer (PBS) at room temperature in a rotary mixer, at an activated-PEG:lysine molar ratio of 75:1. Conditions were standardised so that differences in pH and duration of the coupling reaction could not account for differences in the residual bioactivity. Representative reactions at the same molar ratios were checked by FPLC and found to be ≥85% modified. Modified material was assayed in methylcellulose cultures of normal human bone marrow progenitor cells essentially by the method of Ash et al (1981) Blood, 58 309-316, but substituting recombinant human GM-CSF for leucocyte conditioned medium. Erythroid bursts were scored microscopically on encoded, encrypted dishes on day 14-16 of culture. The results for the optimised TMPEG-derived adducts and for products of the other coupling reactions having the same coupling ratio are shown in Fig. 4 as means of triplicate culture dishes except in Panel A where the results are means ± SEM for 3 independent experiments. Fig 4a shows the excellent conservation of biological activity achieved with EPO PEGylated with the TMPEG (filled circles) compared with unmodified EPO (open circles). Datum points are superimposed except at 4U/ml (33.6 ng/ml) erythropoietin. Similar effects observed in CFUgm assays were found to be due to a modest inhibitory effect of TMPEG in the assay system.

Fig. 4b shows a complete loss of detectable activity when the cyanuric chloride method (filled circles) was used compared with PEG-EPO prepared according to Example 1 (open circles) and a high dose inhibition of endogenously stimulated erythroid colonies. This indicates either the presence of a profoundly inhibitory substance in the reaction mixture or loss of all intrinsic erythropoietic activity in conjunction with some inhibitory material. Lack of even a slight increment in colony formation at low doses and the shallow dose related inhibition of endogenously stimulated colonies at high concentrations favours the latter interpretation. The FPLC profile for this material also differed somewhat from that obtained with the other three activated PEG preparations in that there was a higher proportion of material eluting with the void volume, indicating the presence of more aggregates. This suggests that crosslinking of target molecules may be occurring (as has previously been observed for this agent [Leonard (1984)]).

Fig. 4c shows a somewhat different picture for PEG-EPO prepared by the succinimidyl succinate method (filled circles), with detectable activity only at 4U/ml (33.6ng/ml) compared with PEG-EPO according to the invention (open circles). This type of shift in the dose-response curve is less likely to be due to inhibitor and probably represents over an order of magnitude loss in bioactivity. This is somewhat surprising because this method is claimed to have good conservation of biological activity [Katre (1987), Zalipsky (1992)]. However such claims have been made on the basis of short term assays. In the assay used here the erythropoietin needs to be available throughout the 14-16 day culture period. The cells in these cultures are also a rich source of esterases and the PEG-erythropoietin made by this method contains a potentially labile bond (see Table 1). In this and some related active ester methods, removal of the PEG leaves the molecule modified as follows:- Target-$NH_2$ forms

$$\text{Target-NH-CO-CH}_2\text{-(CH}_2)_n\text{-CO-PEG}$$

then esterase cleavage forms

$$\text{Target-NH-CO-CH}_2\text{-(CH}_2)_n\text{-COOH.}$$

This process might well influence bioactivity adversely since there is a charge conversion from a lysine amino group to a carboxyl group at the previously PEGylated site.

Fig. 4d shows PEG-erythropoietin made using monomethoxy PEG-p-nitrophenylcarbonate (filled circles) compared with unmodified (sham-treated) EPO (open circles). Here there are increments in colony number at low doses similar to that seen with the tresyl chloride activated PEG, but the high-dose inhibition of colony formation is much more evident, indicating the presence of inhibitory material.

Comparative Example 5

An attempt was made to make PEG-erythropoietin using the carbonyldiimidazole method under comparable conditions to those used above, but on this time scale and coupling ratio the degree of modification achieved was not significant.

Example 5

A solution (50µl) of amphotericin (Fungizone) in distilled water (20 mg/ml) was mixed with HEPES buffer (450µl, pH 9.0) containing TMPEG produced according to Example 1 (60 mg/ml). The mixture was then incubated at room temperature for 24 h. As a control, amphotericin was similarly treated but with MPEG substituted for TMPEG. To demonstrate the covalent linkage of the TMPEG to the amphotericin, the partition coefficient of amphotericin in both samples was measured as follows: 100µl samples were mixed with 1 ml of top and 1 ml of bottom phases of a biphasic system containing 5% dextran T-500, 5% PEG-6000, 0.15 M sodium chloride and 0.01 M sodium phosphate pH 6.8. After shaking for 1 min. the system was left to settle for 20 min. and then 500µl from the top and bottom phases were removed to quantify amphotericin (by measuring the absorbance at 330 nm). The % material in the top (PEG-rich) phase was: a) control 4.9 ± 0.2%; b) MPEG control 4.1 ± 0.4%; c) TMPEG (preparation 1) 92.8 ± 0.1%; d) TMPEG (preparation 2) 92.7 ± 0.2%.

Example 6

As an example of creation of an affinity ligand PEG-WGA (polyethyleneglycol modified wheat germ agglutinin) was constructed in exactly the same manner as used in Example 1 and used to bind rightside-out vesicles from human erythrocyte membranes thus altering their partition in a PEG-dextran aqueous two-phase system. Rightside-out vesicles exposed to phosphate buffered saline prior to partitioning showed the following distribution: 17.6 ± 1.2% to the top phase, 48.7 ± 9.0% to the interface and 33.6 ± 8.6% to the bottom phase (mean ± SEM, n=3). Rightside-out vesicles exposed to the PEG-WGA ligand prior to partioning showed the following distribution: 30.1 ± 1.2% to the top phase, 32.7 ± 1.4% to the interface and 7.2 ± 0.6% to the bottom phase. This change in partitioning can be exploited to remove rightside-out vesicles from a mixture with inside-out vesicles thus obtaining a pure preparation of inside-out vesicles from human erythrocyte membranes.

Example 7

Aliquots of human erythrocytes (50μl containing 0.95 x $10^7$ cells) were added to one of the following reagents (500μl):

a) sodium phosphate buffer (0.05M, pH 7.5) containing sodium chloride (0.125M) (PBS).
b) TMPEG freshly prepared as in Example 1 in PBS (36 mg/ml, equivalent to 1.9 ng/cell).
c) TMPEG (36 mg/ml) inactivated with lysine (Sigma Ltd. 4mg/ml) at 2.5:1 molar lysine:TMPEG for 2h at room temperature.

After 30 min incubation at 37°C cells were pelleted down and resuspended in PEG-rich top phase (1 ml) so that their PEGylation could be monitored in a PEG-dextran phase system. Cells incubated in PBS partitioned with 26.2 ± 11.8% (mean ± SD) cells in the top phase. With TMPEG exposure of cells this increased to 72.1 ± 10.7%, whereas with prior inactivation of TMPEG to prevent coupling to cells there was only 32.7 ± 9.3% in the top phase, indicating that covalent attachment of the PEG to the erythrocytes was responsible for the change in partitioning behaviour. The erythrocytes remained intact in the TMPEG-treated and control experiments indicating that the method does not disrupt the cell membrane.

Example 8 and Comparative Example 6

Liposomes were reacted with TMPEG prepared by the process of WO90/04384 (Comparative Example 6) and the process of Example 1 (Example 8). The success of the PEGylation was followed in a PEG-dextran phase system by quantitating the liposome content of the top (PEG-rich) and bottom (dextran-rich) phases and at the interface. Because the TMPEG prepared by the process of Example 1 was known to be more active, only half the molar ratio was used. Despite this, a much higher degree of substitution of the liposome was achieved. This greater reactivity has the advantage that the liposomal preparation does not have to be exposed to so much activated PEG (the level of the latter is limited by its effect on liposomal stability and/or aggregation). 100 nm diameter unilamellar vesicles of bovine spinal cord phosphatidylserine (PS) were prepared by extruding lipid dispersions of PS (10 mg/ml in HEPES buffer pH9), spiked with $^3$H-dipalmitoylphosphatidylcholine through 100 nm polycarbonate filters ten times using an extruder device (Lipex Biomembranes, Canada). Vesicles (300 μl) were incubated with TMPEG (39 mg, molar ratio TMPEG:PS at outer surface 4:1 or 19.5 mg, molar ratio. 2:1) in HEPES buffer (60 μl) for 18 hrs at room temperature. As a control MPEG was substituted for TMPEG. To demonstrate the covalent attachment of PEG to the outer surface of the vesicles the distribution of the vesicle in a two-phase system was measured as follows: samples (20 μl) were mixed with top phase (1 ml) and bottom phase (1 ml) of a biphasic system containing dextran T-500 (5%) and PEG 6000 (5%), sodium chloride (0.15M) and sodium phosphate buffer (0.01M) prepared at 25°C. After shaking for 1 min, triplicate samples (50 μl) were removed for total counts. After the phases had separated at 25°C for 20 min triplicate samples were taken from the top phase (50 μl) and the bottom phase (20 μl) for scintillation counting. Vesicle partitionings, calculated as the percentage of added vesicles that were present in the top(T) and bottom(B) phases and at the interface (100-T-B) were as shown in Table 2.

Table 2

| TMPEG sample | TMPEG:PS Molar ratio | Partitioning (%) (mean of triplicate partitions) | | |
|---|---|---|---|---|
| | | Top | Interface | Bottom |
| Old Method | 4:1 | 6.3±0.6 | 25.3±4.0 | 68.4±3.7 |
| Control MPEG | | 5.5±0.3 | 9.1±4.9 | 85.4±4.8 |
| New Method | 2:1 | 71.6±1.2 | 11.1±1.2 | 17.3±0.5 |
| Control MPEG | | 4.9±0.2 | 1.6±1.6 | 104.3±7.3 |
| | | | | |

The increase in interface partitioning with associated decrease in bottom phase partitioning with the old method indicated some PEGylation. However with the activated PEG made by the new method, even at the lower molar ratio there is extensive partitioning to the top phase indicating the highest degree of PEGylation of the two samples.

To increase the PEGylation with the rather unreactive old method sample, the molar ratio was increased to 20:1, using a final concentration of 35% w/w TMPEG. This caused the vesicles to aggregate. Controls with MPEG also showed aggregation. Addition of 75µl of the reaction mixture to 2.2 ml of N-tris-(hydroxymethyl)methyl-2-aminoethanesulphonic acid (TES) buffet [Fisher et al (1991) Cell and Model Membrane Interactions p.47 Plenum Press, New York] pH 7.4 gave solutions with $OD_{650}$ of 0.26 whereas untreated vesicles in control buffer had $OD_{650}$ of 0.01, indicating that the aggregation observed with the TMPEG and MPEG was not completely reversible. It is well established that PEG solutions can produce fusion and/or permeability increases [Tilcock & Fisher (1982) Biochimica et Biophysica Acta, **688** 645-652, Aldwinkle et al., (1992) ibid, 689: 548]

Example 9

(a) 1µg of the supercoiled plasmid pBR322 was digested with 100 units of the restriction enzyme Rsa I for two hours at 37°C, in low salt buffer consisting of 10mM Tris HCl pH 7.5, 10mM $MgCl_2$, 1 mM dithiothreitol **(DTT)**. This yields four blunt ended fragments per molecule of pBR322. Protein was removed by phenol/chloroform extraction. After ethanol precipitation the DNA was resuspended in a small volume of tris-EDTA **(TE)** pH 7.6 and the DNA was end labelled with [γ32P] dATP using a BCL kit as per the manufacturer's instructions. Removal of the free [$^{32}$P] dATP was achieved using Sephadex G-50 columns. The DNA was then split into 6 aliquots. Three of these aliquots were made single stranded by boiling and then exposed to either TMPEG or MPEG, at a concentration of 400mg/ml in coupling buffer (consisting of 0.005M sodium phosphate 0.125M sodium chloride pH 7.5) or to coupling buffer only, at a volume ratio of 150 µl:150µl), for 20 minutes. Three other aliquots, which were allowed to remain double stranded, were incubated with either TMPEG, MPEG or coupling buffer only, under identical conditions. After incubation, all the samples were again made single stranded by boiling. The samples were examined in a 2% alkaline agarose gel, (50mM NaOH, 1mM EDTA pH 8.0) using alkaline loading buffer. This was a 6x stock consisting of 0.3M NaOH, 6mM EDTA pH 8.0, 18% Ficoll 400, and 0.15% bromocresol green. The samples were run overnight at 50v. The dried gel (Fig. 5) was analysed by autoradiography, the film was developed in an automated Fuji X-ray film developer. Lanes 1-6 from left to right are: 1-3 DNA exposed while double stranded; 4-6 DNA exposed while single stranded; 1&4 coupling buffer; 2&5 MPEG; 3&6 TMPEG. Only single stranded DNA exposed to TMPEG showed a mobility shift demonstrating coupling of PEG to the DNA.

(b) Eco RI digested pBR322 containing a single cleavage site yielding one double stranded fragment with a 3' recessed 4bp cut and the following single stranded overhangs:-

5'NC......AATTCN3'

3'NGTTAA......GN5'.

Of three aliquots, one was treated with a reaction mixture consisting of TMPEG (400 mg/ml) in coupling buffer (50 ml), DNA (circa 0.1 µg in 50 ul of tris EDTA). The coupling buffer (pH 7.5) was a solution of sodium chloride (0.125M) and sodium phosphate (0.05M). The reaction was conducted at room temperature for 20 min. A second aliquot was similarly teated with TMPEG (40 mg/ml) in coupling buffer and the third was treated with coupling buffer alone.

(c) Bam HI digested pBR322 containing a single cleavage site yielding one double stranded fragment with a 3' recessed 4bp cut and the following single stranded overhangs:-

5'NG......GATCCN3'

3'NCCTAG......GN5'.

This material was divided into three aliquots which were treated with two concentrations of TMPEG and coupling buffer as in (b).

To detect the extent of PEG-modification the staggered ended DNA was end-labelled with $^{32}P$ and then partitioned in a PEG-phosphate phase system to determine the partition coefficient (K), results being shown in Table 3.

Table 3

| | No PEG | TMPEG 40mg/ml | TMPEG 400mg/ml |
|---|---|---|---|
| DNA digest | EcoR1 50µg/ml | ECoR1 50µg/ml | ECoR1 50µg/ml |
| PEG-phase (%)* | 41.7 | 58.4 | 96.5 |
| $PO_4$-phase (%)* | 58.3 | 41.5 | 3.5 |
| log(K) | -0.145 | -0.148 | 1.44 |
| DNA digest | BamH1 50µg/ml | BamH1 50µg/ml | BamH1 50µg/ml |
| PEG-phase (%)* | 41.7 | 51.0 | 69.4 |
| $PO_4$-phase (%)* | 58.3 | 49.0 | 30.6 |
| log(K) | -0.145 | -0.017 | 0.356 |

* Yields corrected for differential recovery from the phases via recovery controls.

Up to saturation there is known to be a linear relationship between the number of PEG molecules attached per target molecule and log(K). The DNA cleaved with EcoRI (with two free amino groups supplied by two adenine bases) is more readily modified than the DNA cleaved with BamHI (with three free amino groups supplied by adenine, guanine and cytosine).

<u>Example 10</u>

BALB/c, male, 6-8 week-old mice, weighing approximately 20g, were pooled into groups of three per condition, with *adlib* access to food and water. PEG-modified GM-CSF samples were prepared as in Example 1, using the concentrations of GM-CSF and TM-PEG indicated in Table 4a. The reaction products were evaluated by FPLC and the proportions of individual species were estimated by fitting summated Gaussian curves to the FPLC profiles and estimating the area under the curve corresponding to each individual peak and expressing this as a % of the total area for the whole FPLC profile (Table 4b). The inventors have previously established that the individual peaks seen at low TMPEG concentrations (peaks 0, 1, 2 and 3) correspond to GM-CSF, $PEG_1$-GM-CSF, $PEG_2$-GM-CSF, $PEG_3$-GM-CSF, respectively. At high TMPEG concentrations the peaks are broader and less well resolved thus fitting of individual Gaussian curves over all regions of the FPLC profile may not be possible. Thus peak 3 and the peaks for more highly substituted GM-CSF (potentially $PEG_{4-7}$-GM-CSF) have been pooled. The peak eluting with the void volume peak is well resolved from the $PEG_{4-7}$-GM-CSF and presumably represents aggregates, the area of this peak was used to estimate the proportion of aggregated material (Table 4b).

The different PEGylated samples and unmodified GM-CSF (Hoechst), all containing <4% [$^{125}$I]GM-CSF (Amersham, 1222 Ci/mmol) to serve as a tracer, were adminstrated at a dose of 0.0524µg in 100µl (3.45 x $10^{-12}$ mole; equivalent to 2.5µg/kg body weight) by subcutaneous injection. The amount of TMPEG concomitantly administered is also indicated in Table 4a (MPEG has been shown not to influence $t_{1/2}$, but does influence egress from the subcutaneous site). Up to eight 25µl tail vein blood samples were collected from each mouse using capillary tubes at various time points ranging between 10 minutes and 80 hours, to give a minimum of three samples per time point. Samples from the capillary tubes were washed immediately into 1ml of PBS containing 50 I.U. of heparin. Radioactivity was determined using a gamma counter (LKB 1270 Rackgamma) results being shown in Fig. 6a (open circles for untreated material, other symbols as indicated on the figure). Clearances for the materials of reactions A and B were clearly biphasic, presumably reflecting the large proportion of unmodified material remaining in these preparations (Table 4b). Half-lives were calculated using linear regression (log concentration versus time) for the slower clearing component if biphasic elimination was present (solid lines Figure 6a; dotted lines give the 95% confidence intervals). The $t_{1/2}$ values ±SE are given in Table 5.

With the exception of the product of reaction D, which is known to contain >50% of aggregates (the latter are known to clear slowly), $t_{1/2}$ for the other reactions (A-C) are similar. Although there may be some increment in $t_{1/2}$ between reactions A and B, surprisingly, there is no additional increment in $t_{1/2}$ betwen reactions B and C. This was not anticipated in view of the fact that reaction C produced a higher proportion of $PEG_2$-GM-CSF and adducts with higher PEGylation ratios. The difference in $t_{1/2}$ between reactions A and B is also not marked and, with the proviso that dissection of early and late components introduces more error into the $t_{1/2}$ estimates, it can be inferred that the addition of the first PEG molecule has the greatest impact on the clearance of GM-CSF. This is unexpected because the estimates for apparent molecular weight from FPLC were initially: GM-CSF 14.5kDa; $PEG_1$-GM-CSF 40.5kDa; $PEG_2$-GM-CSF 67.6 kDa; $PEG_3$-GM-CSF 112.9 kDa and after 3 or 4 independant estimates the values were: GM-CSF 12.5 ± 1 kDa; $PEG_1$-GM-CSF 41.5 ± 3.6 kDa; $PEG_2$-GM-CSF 75.2 ± 10.5 kDa; $PEG_3$-GM-CSF 138.5 ± 27.3 kDa. Thus, $PEG_1$-GM-CSF should not exceed the renal molecular weight threshold for excretion and the major increment in the $t_{1/2}$ would have been expected to be between $PEG_1$- and $PEG_2$-GM-CSF not between GM-CSF and $PEG_1$-GM-CSF. This result is also surprising in that the increment in apparent molecular weight is unexpectedly large for the addition of the first MPEG 5000 molecule (12.5 to 41.1 kDa). Other proteins (e.g. recombinant erythropoietin) have shown a surprisingly small increment in apparent molecular weight with the addition of one MPEG 5000 molecule (eg circa 1.4 kDa increment).

In order to establish the contributions to extended $t_{1/2}$ we examined the tissue distribution of material prepared in an identical manner to reaction C, using an identical dose. At each of the times indicated, one aminal was sacrificed and organs/tissue including kidney, spleen, liver, lung, heart and femur were recovered and radioactivity was measured as above. Figure 6b shows the results. The dotted and dashed lines indicate the blood levels of GM-CSF and PEG-GM-CSF respectively. The filled circles and open circles are the organ concentrations of PEG-GM-CSF and GM-CSF respectively. Comparison of organ:blood concentration ratios indicates that renal accumulation (the kidney is the only organ which achieves high organ:blood ratios and is the site which is known to catabolise the peptide to small fragments), is substantially reduced by PEGylation of the degree achieved in reaction C. This is somewhat surprising since circa 24% of the reaction C mixture represents $PEG_1$-GM-CSF (i.e. material below the renal threshold). It suggests that the size of PEG conjugates is not the only factor in reduction of renal clearance. Thus the degree of substitution desirable for individual targets will have to be assessed on a case-by-case basis.

Table 4(A)

| Coupling Ratios and Compositions for PEGylated GM-CSF Preparations | | | | |
|---|---|---|---|---|
| **PREPARATION** | **GM-CSF (µg/ml)** | **TMPEG (mg/ml)** | **GM-CSF (dose, µg)** | **TMPEG (dose, mg)** |
| Unmodified Control | 0.524 | 0 | 0.0524 | 0 |
| Reaction A | 0.524 | 6.46 | 0.0524 | 0.646 |
| Reaction B | 0.524 | 19.75 | 0.0524 | 1.975 |

Table 4(A) (continued)

| Coupling Ratios and Compositions for PEGylated GM-CSF Preparations | | | | |
|---|---|---|---|---|
| **PREPARATION** | **GM-CSF (μg/ml)** | **TMPEG (mg/ml)** | **GM-CSF (dose, μg)** | **TMPEG (dose, mg)** |
| Reaction C | 0.524 | 197.5 | 0.0524 | 19.75 |
| Reaction D | 0.524 | 459.7 | 0.0524 | 45.97 |

Table 4(B)

| Percentage of GM-CSF in different fractions at various coupling reactions. | | | | | |
|---|---|---|---|---|---|
| | **Unmodified GM-CSF (Peak 0)** | **PEG-modified-GM-CSF** | | | |
| | | **Peak-1** | **Peak-2** | **Peak-3 & higher** | **Putative Aggregates** |
| Unmodified Control | 100 | 0.0 | 0.0 | 0.0 | 0.0 |
| Reaction A | 48.6 | 27.7 | 14.1 | 3.1 | 0.0 |
| Reaction B | 31.0 | 26.0 | 21.6 | 8.0 | 1.6 |
| Reaction C | 16.8 | 23.5 | 15.9 | 19.2 | 20.7 |
| Reaction D | 11.1 | 37.7* | | | 51.2 |

* Combined data for all PEG-GM-CSF Peaks (incompletely resolved)

Table 5

| Half-life of late clearing components in PEGylated (GM-CSF) preparations). | | | |
|---|---|---|---|
| | **$t_{1/2}$ (hours)** | **$t_{1/2}$ + SE*** | **$t_{1/2}$ -SE*** |
| Unmodified Control | 1.63 | 1.81 | 1.47 |
| Reaction A | 11.7 (7.2) | 14.0 (8.6) | 10.1 (6.2) |
| Reaction B | 18.1 (11.1) | 23.1 (14.2) | 14.8 (9.1) |
| Reaction C | 16.8 (10.3) | 17.3 (10.6) | 16.4 (10.1) |
| Reaction D | 25.1 (15.3) | 26.0 (15.9) | 24.2 (14.8) |

* calculated from the slope of the regression of log concentration versus time over portions of dose response curves (shown in fig.6(a)), ± the standard error of the slope.
Figures in parentheses indicate the fold increase over the control figure.

Example 11.

A direct comparison using a 2h. coupling period and a molar ratio of activated PEG:lysine of 305:1 gave the following results (assessing modification by phase partitioning as described by Delgado (1990)) is shown in Table 6.

Table 6

| PEGYLATION METHOD | PARTITION COEFFICIENT |
|---|---|
| Unmodified Control | 1.54±0.07 |
| The present invention (TMPEG) | 4.77±0.04 |
| Cyanuric Chloride | 9.0±0.74* |
| Carbonyldiimidazole | 2.06±0.14 |
| Phenylchloroformate | 4.92±0.02 |
| Succinimidyl succinate | 3.35±0.25 |

* Result compounded by the presence of large amounts of cross-linked products for which log K will not be a linear function of modification.

These results demonstrate that a fast reaction rate is achieved with the present invention so that there is less time for a target material to be damaged by the possibly hostile environment of the coupling reaction.

Example 12

After storage at 4°C, $^{125}$I-GM-CSF shows degradation with the appearance of label in low molecular weight material. This process is slowed by PEG modification. Fig.7 shows FPLC profiles of representative examples stored for 1 day (upper panels) and 33 days (lower panels). When supplied, $^{125}$I-GM-CSF has little or no low molecular weight labelled species. After storage for 1 day of an unmodified (open circles) preparation and a modified preparation (exposed to TMPEG at a molar ratio of 305:1 as described above, filled circles) there was a small amount of labelled low molecular weight material (peak 3) in both preparations. In both, the majority of the material (peak 1) is high molecular weight (the size distribution difference is commensurate with the PEGylation of the intact protein and the low molecular weight material probably represents free iodine). After storage the low molecular weight peak had increased substantially and a second peak (peak 2) had appeared between it and the peak of unmodified material (lower panel). There has been generation of intermediate and low molecular weight fragments in both samples. The former are commensurate in size with proteolytic cleavage products and the latter either with free iodine or very small fragments (i.e. < circa 1kDa). The relative amounts of $^{125}$I in the samples is listed in the Table 7. Pegylation reduces the loss of high molecular weight species.

Table 7

| | Peak | Before Storage | After 1 Month Storage |
|---|---|---|---|
| GM-CSF | 1 | 76.1 | 9.2 |
| | 2 | 0 | 20.6 |
| | 3 | 21.5 | 68.2 |
| PEG-CM-CSF | 1 | 81.1 | 45.8 |
| | 2 | 0 | 8.9 |
| | 3 | 12.4 | 36.3 |

Example 13

Dry stored TMPEG, prepared as in Example 1 above, derived from an MPEG preparation (Union Carbide UK) contaminated with circa 22% PEG (when estimated by NMR) was used in this Example. This contaminating PEG results (during the polymer activation process) in the formation of biactivated tresyl PEG which produces aggregates in the final product; the formation of aggregates can be reduced by reducing the level of contamination of the MPEG preparation by PEG. The erythropoietin (EPO) preparation used was a commercial preparation obtained from Cilag Ltd and the erythropoietin stock contained 3200 u/ml in PBS. This preparation contains 3200 units of EPO, and 2 mg/ml of human serum albumin. The coupling ratios were calculated on the basis of the total number of available lysine groups in the erythropoietin and carrier protein, and would need to be adjusted accordingly for pure erythropoietin preparations. The erythropoietin preparation was exposed to the stated molar ratios of TMPEG:lysine and was incubated at room temperature for two hours on a rotary mixer. The sham-reacted control was similarly exposed to diluent, (PBS). The resulting samples were analysed by FPLC the profiles being shown in Fig.8 panels A to F; the vertical axis in all cases is C.P.M.[$^{125}$I]-erythropoietin: A) Sham treated;B to F TMPEG:lysine at ratios B)9.375:1; C) 18.75:1; D) 37.5:1; E) 75:1; F) 305:1

Samples (200μl containing circa 10.5μg/ml total proteins) were analysed on a Pharmacia FPLC system with a Superose 12 HR 10/30 column previously equilibrated with PBS. The samples were loaded onto the column and then eluted with sterile PBS at a flow rate of 0.3ml per minute; 0.25ml fractions were collected. To monitor protein profiles, PEG-EPO was prepared using [$^{125}$I]-erythropoietin (Amersham) and fractions were analysed using a gamma counter (Nuclear Enterprises NE1600). To check the biological activity of a series of preparations made using the same TMPEG preparation, over a similar range of molar ratios of TMPEG:lysine, using unlabelled EPO (Cilag), serial dilutions of the unfractionated reaction mixture were exposed to the haemopoietic cell-line, TF-1, to induce thymidine uptake. Cultures were performed in microtitre plates containing 50 μl of cell suspension (5 x $10^4$ cells/well) in RPMI-1640 medium containing 5% foetal calf serum (FCS) in round bottom microwell plates (NUNC). To increase sensitivity of TF-1 cells to cytokine, they were deprived of their maintenance growth stimulus (GM-CSF at 50 units/ml) for 24 hours prior to assay. Each well contained either PEG-erythropoietin or unmodified erythropoietin, diluted in RMPI-1640. Cultures were incubated in a humidified atmosphere of 5% carbon dioxide in air at 37°C for 18 h. Cells were then exposed to 0.5μCi of [methyl$^3$H]thymidine 25Ci/mmol. Amersham) in 50μl of culture medium and incubation was continued as above for 4 h. Cells were harvested onto glass filters (Titertek Flow labs) using a Dynateck multimesh 2000 harvester, washed with methanol and dried for 12 h at room temperature. [$^3$H]-thymidine incorporation was determined by scintillation counting (Beckman LS 5000 CE) using 5ml Filter Count (Packard) the results being expressed as D.P.M. of the test sample minus D.P.M of unstimulated cells; negative values therefore indicate inhibition of background thymidine uptake.

Fig.9a shows the induction of thymidine uptake by all preparations, both PEG-modified and unmodified. Fig. 9b shows the early portion of the dose-response curves for concentrations less than one unit/ml at expanded scale to facilitate comparison. All preparations produced stimulation of incorporation of thymidine, but there is a progressive contamination by inhibitory material, whose activity is most evident at 3 and 10 units/ml of erythropoietin. The inhibitor is related to the TMPEG:lysine molar ratio suggesting that either TMPEG itself or co-product is responsible (TMPEG does have an inhibitory effect in this assay system [Malik (1992)]. This inhibitory material was not evident to a similar degree in PEG-GM-CSF preparations, even those prepared with the same batch of TMPEG, and its nature has not yet been determined but may reflect altered sensitivity of the cell line to inhibition under different growth conditions. The similarity of the upward portion of the dose-response curves indicates that there is little inherent loss of biological activity for all the preparations studied.

<u>Example 14</u>

NMR analysis showed that a standard preparation of MPEG-5000 (kindly donated by Union Carbide) contained 23.5 mole % PEG and 76.5 mole % MPEG. Method: 1H-nmr spectra were acquired on a Brucker WN 259 spectrometer on polymer samples in DMSO-$d_6$ (50mg/ml). Integrations of the peaks at 4.56ppm (OH), 3.56 ppm ($CH_2CH_20$) and at 3.26 ppm ($OCH_3$) were made and used to calculate the PEG and MPEG contents (also the molecular mass of the sample which was 5200Da).

PEG contamination results from its production as a by-product during the polymerisation reaction for the manufacture of MPEG (hydroxide used to catalyse the reaction, initiates a side reaction yielding PEG). Since PEG has two termini at which polymerisation can take place, it should achieve a larger molecular weight than that of the MPEG. These data confirm that this side reaction takes place largely during the early phase of the reaction, since the size distribution of the PEG product is approximately double that of the MPEG product and there is no substantial proportion of material spanning a range of intermediate sizes (as would be anticipated if the side reaction continued throughout the polymerisation).

To confirm that the contaminating PEG is, as anticipated, responsible for aggregate formation during the coupling reaction, a TMPEG preparation derived from the same MPEG preparation analysed above was fractionated on the expectation that it would contain circa 25.5% bi-activated PEG. [Such fractionation requires prolonged exposure to an aqueous environment and resulted in some overall loss of activity (thus requiring appropriate sham-treatment of unfractionated controls) and is therefore not advisable as a step in the process of the invention, rather purification prior to activation is recommended]. The TMPEG was then exposed to $^{125}$I-gm-CSF under standard coupling conditions as described over a range of molar ratios as indicated in Table 8 (aggregate formation is more likely at higher molar ratios and/or concentrations of polymer and target). Aggregates were identified by FPLC on Superose 12 (as labelled material eluting with the void volume) and PEG-modified GM-CSF species (as labelled material eluting faster than unmodified GM-CSF, but not as fast as the aggregated material). This identification has been confirmed independently by phase partitioning. The fractionation by vesicle chromatography separates material less prone to produce aggregates (permeable fraction) and more prone (excluded fraction) than the starting material respectively. This validates two points; first that the PEG becomes biactivated and second that the reduction of the contaminating PEG by size fractionation will reduce he amount of aggregates. Since the acceptable level of aggregates will vary from application to application a limit for contamination of MPEG by PEG will have to be selected on a case-by-case basis.

Table 8

Reduction of PEG contamination reduces aggregate formation. Aggregates are expressed as % total PEG-modified material (aggregates + $PEG_n$-gm-CSF where n=1-7)

Table 8

| | **Unfractionated MPEG** | **Permeable Fraction** | **Excluded Fraction** |
|---|---|---|---|
| PREP 1 (150:1 TMPEG:lysine) | | | |
| Aggregates | 21.6 | 6.1 | N.T. |
| PREP 2 (120:1) TMPEG:lysine) | | | |
| Aggregates | 17.9 | 0 | N.T. |
| PREP 3 (17:1 TMPEG:lysine) | | | |
| Aggregates | 0 | 0 | 13.3 |

N.T. = Not Tested

## Claims

**1.** A process for producing an adduct of a polyethylene glycol (PEG) polymer and a target material which process comprises the steps of

a) reacting tresyl-chloride with a polymer of formula (II)

$$(C)_c - PEG - (OH)_g \qquad (II)$$

wherein

the PEG has a valency c+g,
c is zero or a positive number and
g is a postive number

so as to form

a sulphonate ester-activated polymer of formula (III)

$$(C)_c - PEG - (tresyl)_g \qquad (III)$$

wherein

C is a capping group
c and g are as defined above

(b) reacting the activated polymer of formula (III) with the target material and

(c) recovering the adduct of the polymer and the target material,
in which process:

(i) the polymer of formula (II) is dried as obtainable by distillation of any water-benzene azeotrope and then the benzene from a benzene solution of the polymer and said dried polymer of formula (II) is immediately dissolved in an organic solvent which is inert to the reagents and to the product of formula (III) and is anhydrous as obtainable using molecular sieves of 0.3 nm;

(ii) the polymer solution formed in step (i) is reacted with the tresyl chloride in the presence of a base in a reaction vessel from which water is excluded, the polymer of formula III is obtained as free of base as obtainable by removing the organic solvent and dissolving the resultant solid in methanol/hydrochloric acid (1000:0.3), recovering the polymer of formula III by precipitating overnight, recovering the resultant precipitate and supernatant by centrifugation, repeating the dissolying-precipitating-centrifugation steps until no base is detectable in the supernatant;

(iii) the sulphonate ester-activated polymer of formula (III) so produced is recovered dry as obtainable by liquid nitrogen freezing and lyophilisation under reduced pressure;

(iv) the product of step (iii) is then used directly in step (b) or stored as to avoid hydrolysis prior to use in step (b); and

(v) the reaction in step (b) of the sulphonate ester-activated polymer with the target material is conducted in a non-denaturing medium at non-denaturing temperature with respect to the target material.

**2.** A process according to claim 1 comprising, in step (b), reacting, in predetermined molar ratio and at predetermined concentrations, a sulphonate ester-activated polymer having only one tresyl moiety with a target material having more than one reactive group so as to form an adduct of PEG and target having a preselected number of PEG molecules per molecule of target, or having a preselected proportion of the reactive groups reacted with and linked to PEG molecules, and then recovering the adduct in accordance with step (c).

**3.** A process according to claim 1 comprising, in step (b), reacting, in predetermined molar ratio, and at predetermined concentrations, a sulphonate ester-activated polymer having two or more tresyl sulphonyl moieties -AM with a target material having two or more reactive groups so as to produce a sulphonate ester-activated 1:1 adduct of PEG and target than changing the reaction conditions so as to hinder intermolecular reactions whilst permitting intramolecular reactions between the terminal carbon atom of the PEG and reactive groups on the target so as to form adducts of PEG and target having two or more covalent bonds between the PEG and target moieties, and then recovering the adduct in accordance with step (c).

**4.** A process according to any preceding claim wherein the target, is selected from proteins, peptides, amino acids and their derivatives, antibodies and fragments thereof, , IL-1, IL-2, IL-3, IL-4, IL-5, IL-6, IL-7, IL-8, IL-9, IL-10, IL-11 and other interleukins and subtypes thereof, and other cytokines and derivatives or fragments thereof, granulocyte-macrophage colony stimulating factor, the alpha and beta forms of granulocyte-colony stimulating factor, macrophage-colony stimulating factor, and other colony stimulating factors, erythropoietin, haemopoietin-alpha and kit-ligand and other haemopoietins, IFNalpha, IFNbeta and IFNgamma and other interferons, epidermal growth factor, platelet derived growth factor, transforming growth factor (alpha and beta forms), amphiregulin, somatomedin-C, bone growth factor, fibroblast growth factors, insulin-like growth factors, heparin binding growth factors, tumour growth factors and other growth factors and bifunctional growth modulators, macrophage differentiating factor, differentiation inducing factor (DIF), leukaemia inhibitory factor and other differentiation factors, platelet activating factor, macrophage activation factor, and other activating factors, heparin, proteases and their pro-factors, clotting factors VII, VIII, IX, X, XI and XII, antithrombin III, protein C, protein S, streptokinase, urokinase, prourokinase, tissue plasminogen activator, fibrinogen, hirudin, other fibrinolytic/anticoagulant agents and other coagulation factors, peptide hormones, enzymes, vaccines, transcription factors and transcriptional modulators, carbohydrates, glycosoaminoglycans, glycoproteins and polysaccharides, phosphatidylethanolamine and phosphatidylserine and derivatives thereof, sphingosine, cholesterol and other steroids and derivatives thereof, nucleotides, nucleosides, heterocyclic bases, DNA, RNA, synthetic and non-synthetic oligonucleotides, vitamins, antibiotics, bacteristatic and bactericidal, antifungal, anthelminthic agents, noradrenalin, alpha adrenergic receptor ligands, dopamine receptor ligands, histamine receptor ligands, GABA/benzodiazepine receptor ligands, serotonin receptor ligands, leukotrienes and tri-iodothyronine and other small effector molecules, doxorubicin, methotrexate and other cytotoxic agents and derivatives thereof, large multi-molecular structures, erythrocytes, erythrocyte "ghosts" leukocytes, liposomes such as multilamellar vesicules and unilamellar vesicules, micelles and micelle-like structures, and aggregates, microemulsions, coacervates, emulsions or suspensions thereof.

## Patentansprüche

1. Verfahren zur Herstellung eines Adduktes eines Polyethylenglykol(PEG)-Polymers und eines Zielmaterials mit den Stufen, in denen man

(a) Tresylchlorid mit einem Polymer der Formel (II)

$$(C)_c - PEG - (OH)_g \quad (II)$$

worin

das PEG eine Bindigkeit c + g hat,
c 0 oder eine positive Zahl ist und
g eine positive Zahl ist,

so umsetzt, daß

ein sulfonesteraktiviertes Polymer der Formel (III)

$$(C)_c - PEG - (Tresyl)_g \quad (III)$$

gebildet wird, worin

C eine blockierende Gruppe ist und
c und g wie oben definiert sind,

(b) das aktivierte Polymer der Formel (III) mit dem Zielmaterial umsetzt und
(c) das Addukt des Polymers und des Zielmaterials gewinnt, wobei in diesem Verfahren:

(i) das Polymer der Formel (II) so getrocknet wird, wie es durch Destillation eines Wasser-Benzol Azeotrops und dann des Benzols aus einer Benzollösung des Polymers erhältlich ist, und das getrocknete Polymer der Formel (II) unmittelbar in einem organischen Lösungsmittel aufgelöst wird, welches für die Reagenzien und das Produkt der Formel (III) inert ist und so wasserfrei ist, wie es unter Verwendung von Molekularsieben von 0,3 nm erhältlich ist
(ii) die in Stufe (i) gebildete Polymerlösung mit dem Tresylchlorid in Gegenwart einer Base in einem Reaktionsbehälter, aus welchem Wasser ausgeschlossen wird, umgesetzt wird, das Polymer der Formel (III) so basenfrei erhalten wird, wie durch Entfernung des organischen Lösungsmittels und Auflösen des resultierenden Feststoffes in Methanol/Chlorwasserstoffsäure (1000 : 0,3), Gewinnung des Polymers der Formel (III) durch Ausfällung über Nacht, Gewinnung des resultierenden Niederschlages und des oben Schwimmenden durch Zentrifugieren, Wiederholung der AuflösungsAusfällungs-Zentrifugierstufen, bis eine Base in dem oben Schwimmenden feststellbar ist, erhältlich ist,
(iii) das so erzeugte sulfonatesteraktivierte Polymer der Formel (III) so trocken wie durch Tiefkühlen mit flüssigem Stickstoff und Lyophilisieren unter vermindertem Druck erhältlich ist, gewinnt,
(iv) das Produkt der Stufe (iii) dann direkt in der Stufe (b) verwendet oder so gespeichert wird, daß eine Hydrolyse oder Verwendung vor der Stufe (b) vermieden wird, und
(v) die Umsetzung des sulfonesteraktivierten Polymers in Stufe (b) mit dem Zielmaterial in einem nicht denaturierenden Medium bei nicht denaturierender Temperatur in Bezug auf das Zielmaterial durchgeführt wird.

2. Verfahren nach Anspruch 1, bei dem in der Stufe (b) in einem vorbestimmten Molverhältnis und bei vorbestimmten Konzentrationen ein sulfonesteraktiviertes Polymer mit nur einem Tresylrest mit einem Zielmaterial umgesetzt wird, welches mehr als eine reaktive Gruppe hat, um so ein Addukt von PEG und Zielmaterial mit einer ausgewählten Anzahl von PEG-Molekülen je Zielmaterialmolekül zu bilden, oder einen vorausgewählten Anteil der reaktiven Gruppen mit den PEG-Molekülen umgesetzt und vernetzt besitzt, und dann das Addukt gemäß der Stufe (c) gewonnen wird.

3. Verfahren nach Anspruch 1, bei dem in der Stufe (b) in vorbestimmtem Molverhältnis und bei vorbestimmten Konzentrationen ein sulfonatesteraktiviertes Polymer mit zwei oder mehr Tresylsulfonylresten -AM mit einem Zielmaterial mit zwei oder mehr reaktiven Gruppen so umgesetzt wird, daß ein sulfonatesteraktiviertes 1 : 1-Addukt von PEG und Zielmaterial erzeugt wird, dann die Reaktionsbedingungen so verändert werden, daß intermolekulare Reaktionen verhindert werden, während intramolekulare Reaktionen zwischen dem endständigen Kohlenstoffatom des PEG und der aktiven Gruppen an dem Zielmaterial gestattet sind, um so Addukte von PEG und Zielmaterial mit zwei oder mehr kovalenten Bindungen zwischen dem PEG und den Zielmaterialresten zu bilden, und dann das Addukt gemäß der Stufe (c) gewonnen wird.

4. Verfahren nach einem der vorausgehenden Ansprüche, bei dem das Zielmaterial unter Proteinen, Peptiden, Aminosäuren und ihren Derivaten, Antikörpern und Bruchstücken hiervon, IL-1, IL-2, IL-3, IL-4, IL-5, IL-6, IL-7, IL-8, IL-9, IL-10, IL-11 und anderen Interleukinen und Untertypen hiervon und anderen Cytokinen und Derivaten oder Bruchstücken hiervon, Granulozyt-makrophagkoloniestimulierendem Faktor, den alpha- und beta-Formen von Granulozyt-koloniestimulierendem Faktor, Makrophag-koloniestimulierendem Faktor und anderen koloniestimulierenden Faktoren, Erythropoietin, Hämopoietin-alpha und Kit-Ligand und anderen Hämopoietinen, IFNalpha, IFNbeta und IFNgamma und anderen Interferonen, Epidermalwachstumsfaktor, von Blutplättchen, sich herleitendem Wachstumsfaktor, Wandlungswachstumsfaktor (alpha- und beta-Formen), Amphiregulin, Somatomedin-C, Knochenwachstumsfaktor, Fibroblastwachstumsfaktoren, insulinartigen Wachstumsfaktoren, heparinbindenden Wachstumsfaktoren, Tumorwachstumsfaktoren und anderen Wachstumsfaktoren und bifunktionellen Wachstumsmodulatoren, makrophagdifferenzierendem Faktor, differenzierungsinduzierendem Faktor (DIF), Leukämiehemmfaktor und anderen Differenzierungsfaktoren, blutplättchenaktivierendem Faktor, makrophagaktivierendem Faktor und anderen aktivierenden Faktoren, Heparin, Proteasen und ihren Profaktoren, klumpenbildenden Faktoren VII, VIII, IX, X, XI und XII, Antithrombin XIII, Protein C, Protein S, Streptokinase, Urokinase, Prourokinase, Gewebeplasminogenaktivator, Fibrinogen, Hirudin, anderen fibrinolytischen/antikoagulierenden Mitteln und anderen Koagulationsfaktoren, Peptidhormonen, Enzymen, Vaccinen, Transkriptionsfaktoren und Transkriptionsmodulatoren, Kohlenhydraten, Glycosoaminoglycanen, Glycoproteinen und Polysacchariden, Phosphatidylethanolamin und Phosphatidylserin sowie Derivaten hiervon, Sphingosin, Cholesterol und anderen Steroiden und Derivaten hiervon, Nukleotiden, Nukleosiden, heterozyklischen Basen, DNA, RNA, synthetischen und nicht synthetischen Oligonukleotiden, Vitaminen, Antibiotika, Bakteriostatika und Bakteriziden, Antipilzmitteln, Anthelmintika, Noradrenalin, alpha-adrenergischen Rezeptorliganden, Dopamin-Rezeptorliganden, Histaminrezeptorliganden, GABA/Benzodiazepinrezeptorliganden, Serotoninrezeptorliganden, Leukotrienen und Triiodthyronin und anderen kleinen Effektormolekülen, Doxorubicin, Methotrexat und anderen cytotoxischen Mitteln und Derivaten hiervon, großen multimolekularen Strukturen, Erythrozyten, Erythrozyt-"Geist"-Leukozyten, Liposomen, wie multilamellaren Bläschen und unilamellaren Bläschen, Mizellen und mizellenartigen Strukturen sowie Aggregaten, Mikroemulsionen, Coacervaten, Emulsionen oder Suspensionen hiervon ausgewählt wird.

**Revendications**

1. Procédé de production d'un adduit d'un polymère polyéthylène-glycolique (PEG) et d'une matière cible, procédé qui comprend les étapes consistant :

(a) à faire réagir le chlorure de trésyle avec un polymère de formule (II)

$$(C)_c\text{-PEG-}(OH)_g \qquad \text{(II)}$$

dans laquelle

le PEG a une valence c + g,
c est égal à zéro ou à un nombre positif et
g est un nombre positif

de manière à former

un polymère activé par un ester sulfonique, de formule (III)

$$(C)_c\text{-PEG-}(\text{trésyle})_g \qquad \text{(III)}$$

dans laquelle

C est un groupe protecteur,
c et g sont tels que définis ci-dessus

(b) à faire réagir le polymère activé de formule (III) avec une matière cible et
(c) à recueillir l'adduit du polymère et de la matière cible,
procédé dans lequel :

(i) le polymère de formule (II) est séché de la manière qu'on peut l'obtenir par distillation de tout azéotrope eau-benzène puis du benzène d'une solution benzénique du polymère et le polymère séché de formule (II) est dissous immédiatement après dans un solvant organique qui est inerte vis-à-vis des réactifs et vis-à-vis du produit de formule (III) et qui est anhydre comme on peut l'obtenir en utilisant des tamis moléculaires de 0,3 nm ;
(ii) la solution de polymère formée dans l'étape (i) est amenée à réagir avec le chlorure de trésyle en présence d'une base dans un récipient de réaction dont l'eau est exclue, le polymère de formule (III) est obtenu sous forme exempte de base comme on peut l'obtenir en éliminant le solvant organique et en dissolvant la matière solide résultante dans un mélange méthanol / acide chlorhydrique (1000 : 0,3), isolement du polymère de formule (III) par précipitation pendant une nuit, isolement du précipité résultant et du liquide surnageant par centrifugation, répétition des étapes de dissolution - précipitation - centrifugation jusqu'à ce qu'on ne puisse plus détecter de base dans le liquide surnageant ;
(iii) le polymère activé par l'ester sulfonique de formule (III) ainsi produit est recueilli à l'état sec tel qu'on peut l'obtenir par congélation dans de l'azote liquide et lyophilisation sous pression réduite ;
(iv) le produit de l'étape (iii) est ensuite utilisé directement dans l'étape (b) ou conservé de manière à éviter une hydrolyse avant l'utilisation dans l'étape (b) ; et
(v) la réaction dans l'étape (b) du polymère activé par un ester sulfonique avec la matière cible est conduite dans un milieu non dénaturant à une température non dénaturante vis-à-vis de la matière cible.

**2.** Procédé suivant la revendication 1, comprenant, dans l'étape (b), la réaction, dans un rapport molaire prédéterminé et à des concentrations prédéterminées, d'un polymère activé par un ester sulfonique portant un seul groupement trésyle avec une matière cible portant plus d'un groupe réactif de manière à former un adduit de PEG et de la cible ayant un nombre prédéterminé de molécules de PEG par molécule de cible, ou ayant une proportion prédéterminée des groupes réactifs ayant réagi avec des molécules de PEG et s'étant liée à ces molécules, puis l'isolement de l'adduit conformément à l'étape (c).

**3.** Procédé suivant la revendication 1, comprenant, dans l'étape (b), la réaction, dans un rapport molaire prédéterminé et à des concentrations prédéterminées, d'un polymère activé par un ester sulfonique portant deux ou plus de deux groupements sulfonyle de trésyle -AM, avec une matière cible portant deux ou plus de deux groupes réactifs de manière à produire un adduit à 1:1, activé par un ester d'acide sulfonique, de PEG et de cible puis le changement des conditions réactionnelles de manière à empêcher des réactions intermoléculaires tout en permettant des réactions intramoléculaires entre l'atome terminal de carbone du PEG et des groupes réactifs portés par la cible afin de former des adduits de PEG et de cible présentant deux ou plus de deux liaisons covalentes entre les groupements PEG et cible, puis l'isolement de l'adduit conformément à l'étape (c).

**4.** Procédé suivant l'une quelconque des revendications précédentes, dans lequel la cible est choisie entre des protéines, des peptides, des amino-acides et leurs dérivés, des anticorps et leurs fragments, IL-1, IL-2, IL-3, IL-4, IL-5, IL-6, IL-7, IL-8, IL-9, IL-10, IL-11 et d'autres interleukines et sous-types d'interleukines, et d'autres cytokines et leurs dérivés ou fragments, le facteur stimulant la formation de colonies de granulocytes ou de macrophages, les formes alpha et bêta du facteur stimulant la formation de colonies de granulocytes, le facteur stimulant la formation de colonies de macrophages et d'autres facteurs stimulant la formation de colonies, l'érythropoïétine, l'hémopoïétine-alpha et kit-ligand et d'autres hématopoïétines, l'IFNalpha, IFNbêta et l'IFNgamma et d'autres interférons, le facteur de croissance épidermique, le facteur de croissance dérivé des plaquettes, le facteur de croissance transformant (formes alpha et bêta), l'amphiréguline, la somatomédine-C, le facteur de croissance osseuse, les facteurs de croissance des fibroblastes, les facteurs de croissance analogues à l'insuline, les facteurs de croissance de liaison à l'héparine, les facteurs de croissance des tumeurs et d'autres facteurs de croissance et des modulateurs bifonctionnels de croissance, le facteur de différenciation des macrophages, le facteur induisant la différenciation (DIF), le facteur inhibiteur de leucémie et d'autres facteurs de différenciation, le facteur d'activation des plaquettes, le facteur d'activation des macrophages et d'autres facteurs d'activation, l'héparine, les protéases et leurs pro-facteurs, les facteurs de coagulation VII, VIII, IX, X, XI et XII, l'antithrombine III, la protéine C, la protéine S, la streptokinase, l'urokinase, la prourokinase, l'activateur tissulaire du plasminogène, le fibrinogène, l'hirudine, d'autres agents fibrinolytiques / anti-coagulants et d'autres facteurs de coagulation, des hormones peptidiques, des enzymes, des vaccins, des facteurs de transcription et des modulateurs trancriptionnels, des glucides, des glycosoaminoglycanes, des glycoprotéines et des polysaccharides, la phosphatidyléthanolamine et la phosphatidyl-sérine et leurs dérivés, la sphingosine, le cholestérol et d'autres stéroïdes et leurs dérivés, des nucléotides, des nucléosides, des bases hétérocycliques, l'ADN, l'ARN, des oligonucléotides synthétiques et non synthétiques, des vitamines, des antibiotiques, des agents bactériostatiques et bactéricides, antifongiques, anthelminthiques, la noradrénaline, des ligands de récepteurs alpha-adrénergiques, des ligands de récepteurs de dopamine, des ligands de récepteurs d'histamine, des ligands de récepteurs d'acide gamma-aminobutyrique / benzodiazépine, des ligands de récepteurs de sérotonine, des leucotriènes et la tri-iodothyronine et d'autres petites molécules effectrices, la doxorubicine, le méthotrexate et d'autres agents cytotoxiques et leurs dérivés, de grandes structures multimoléculaires, des érythrocytes, des leucocytes "fantômes" érythrocytaires, des liposomes tels que des vésicules multilamellaires et des vésicules unilamellaires, des micelles et structures micellaires, et leurs agrégats, microémulsions, coacervats, émulsions ou suspensions.

Fig.1a.

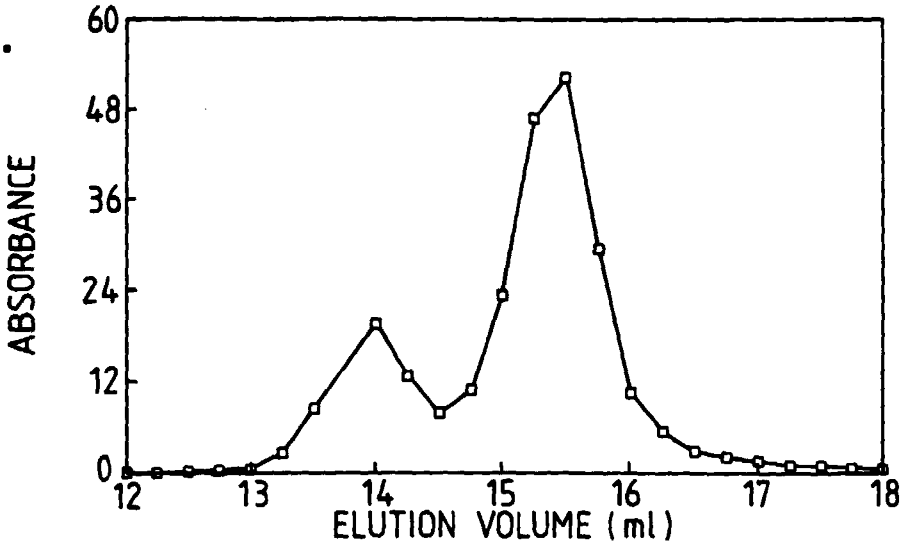


Fig.1b.

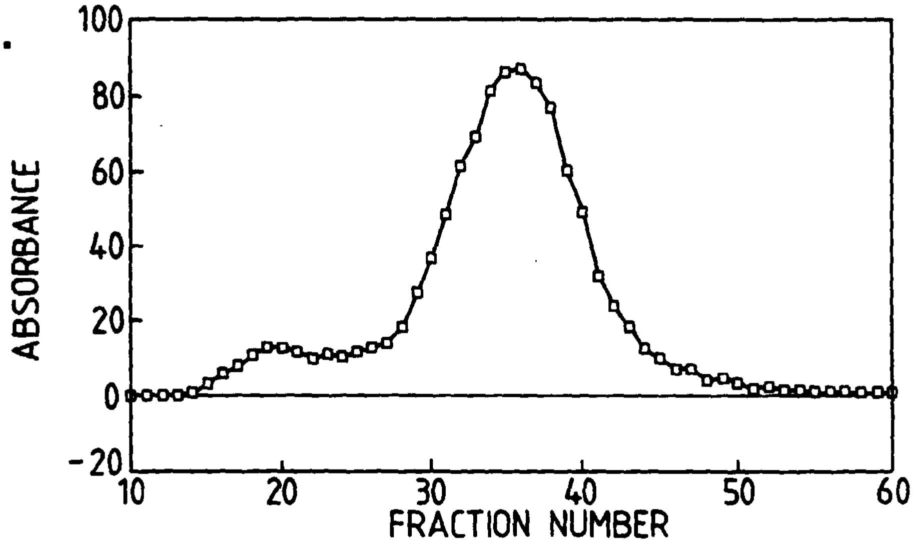


Fig.1c.

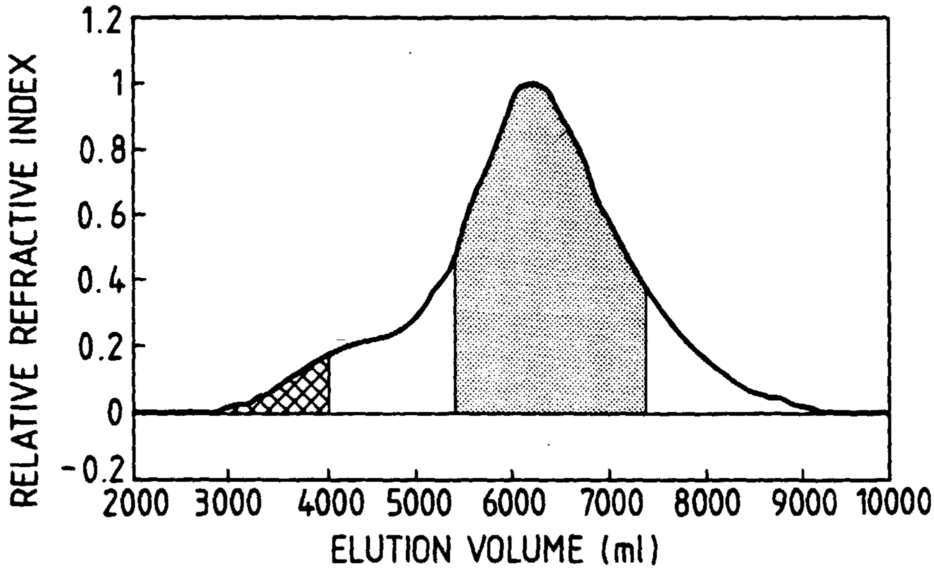

Fig.1d.

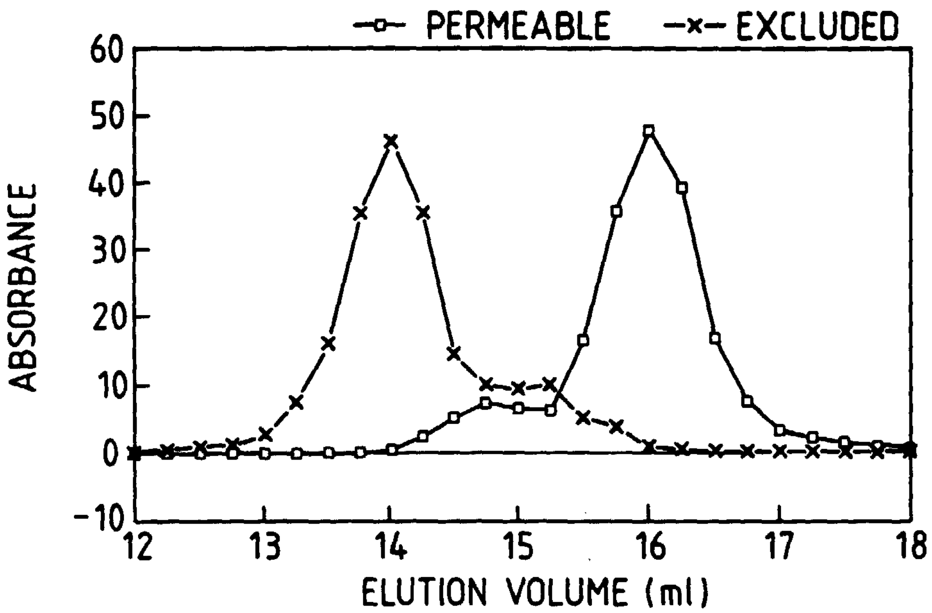
PERMEABLE
EXCLUDED
ABSORBANCE
60
50
40
30
20
10
0
-10
12
13
14
15
16
17
18
ELUTION VOLUME (ml)

Fig.1e.

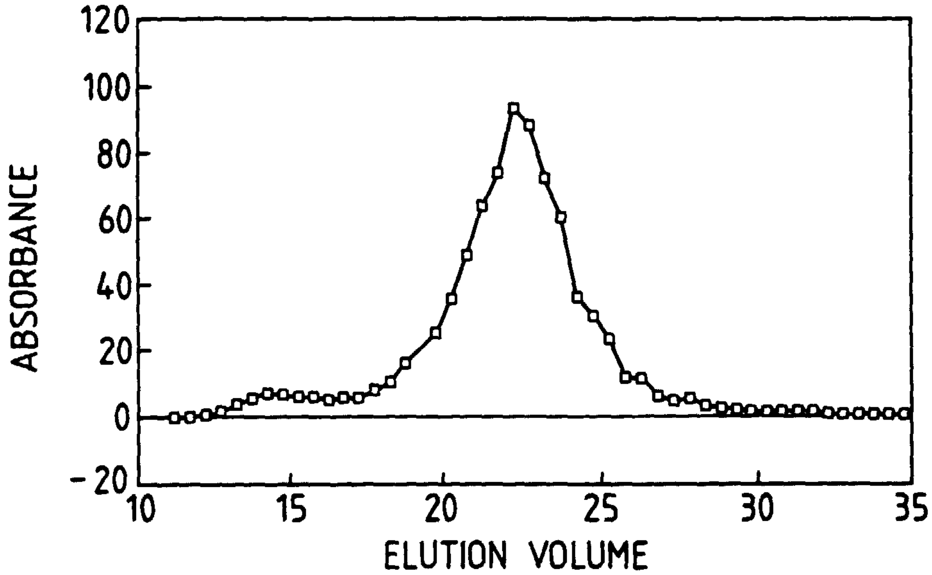
ABSORBANCE
120
100
80
60
40
20
0
-20
10
15
20
25
30
35
ELUTION VOLUME

Fig.2.

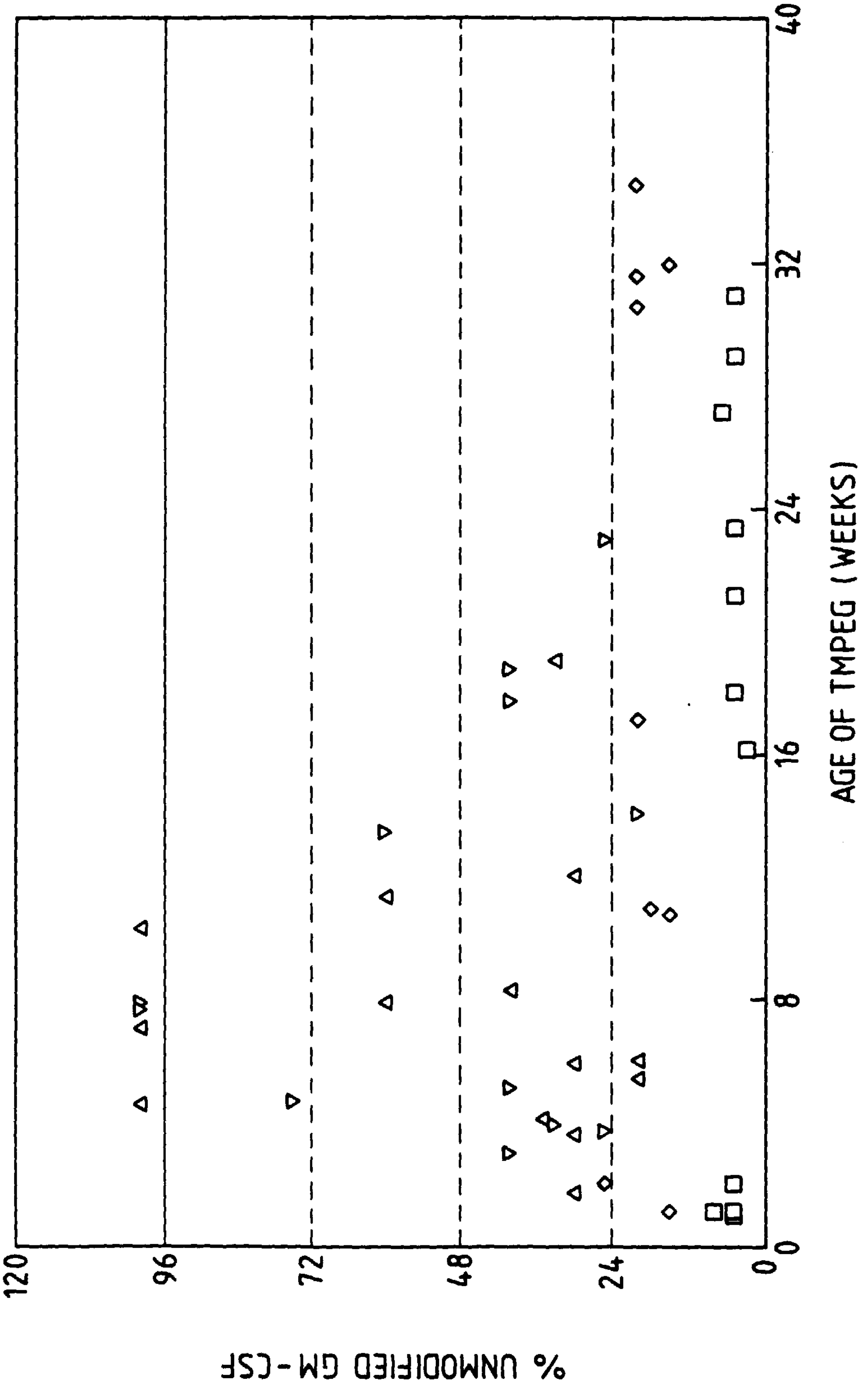
% UNMODIFIED GM-CSF
0
24
48
72
96
120
AGE OF TMPEG (WEEKS)
0
8
16
24
32
40

Fig.3a.

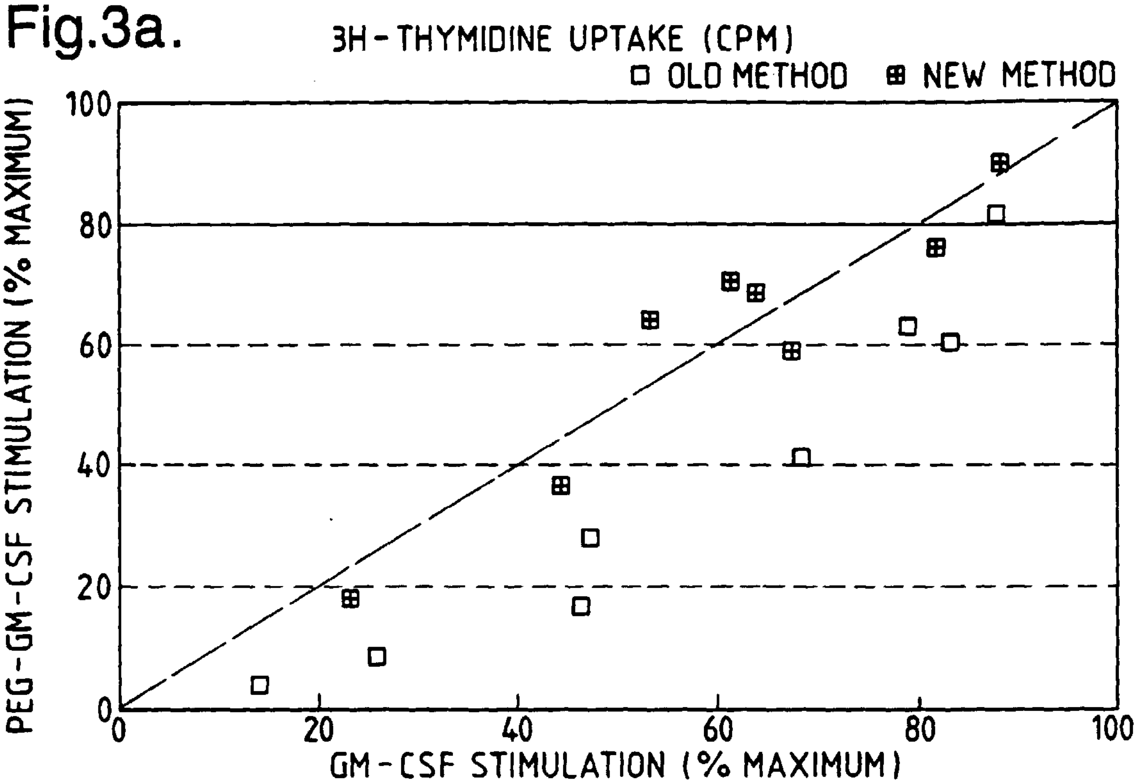
3H-THYMIDINE UPTAKE (CPM)
OLD METHOD
NEW METHOD
PEG-GM-CSF STIMULATION (% MAXIMUM)
GM-CSF STIMULATION (% MAXIMUM)
0
20
40
60
80
100

Fig.3b.

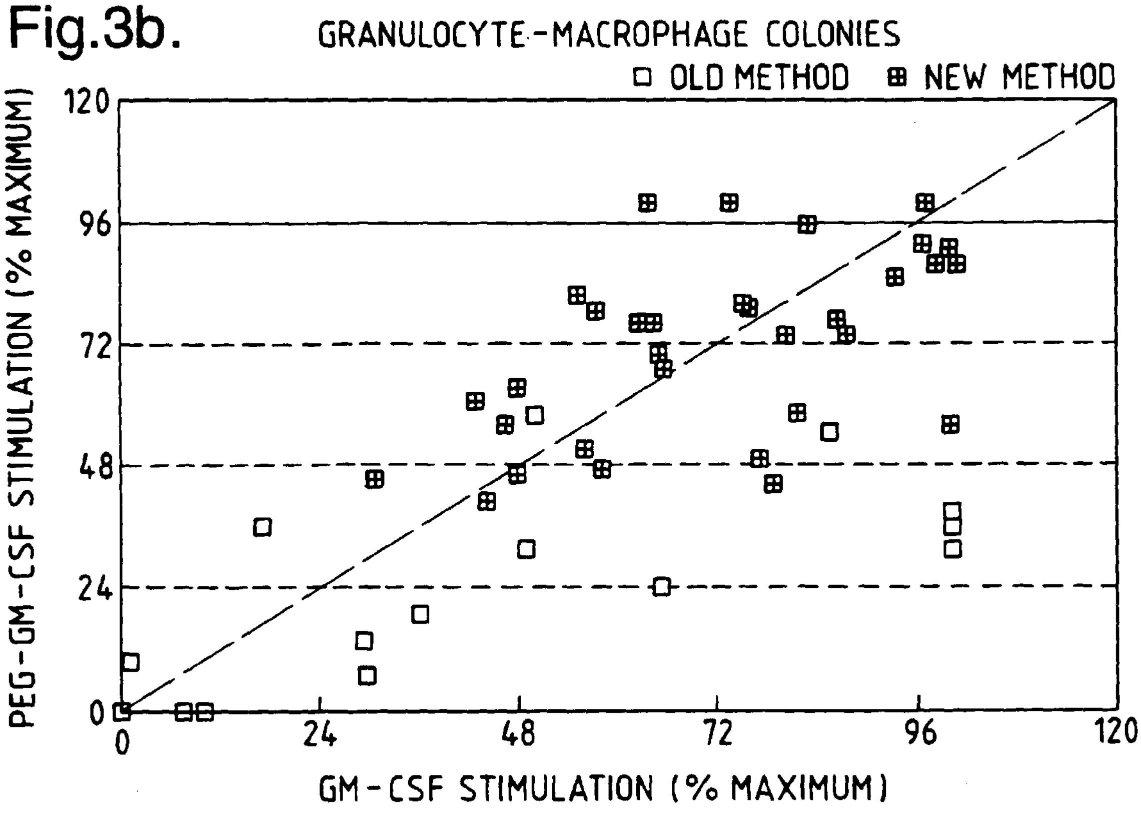
GRANULOCYTE-MACROPHAGE COLONIES
OLD METHOD
NEW METHOD
PEG-GM-CSF STIMULATION (% MAXIMUM)
GM-CSF STIMULATION (% MAXIMUM)
0
24
48
72
96
120

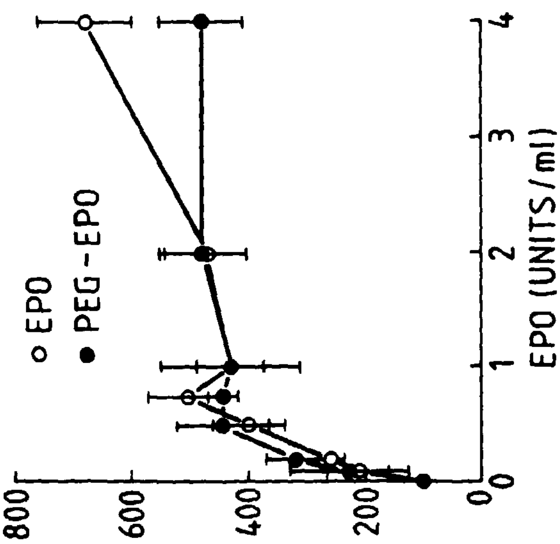


Fig.4a.

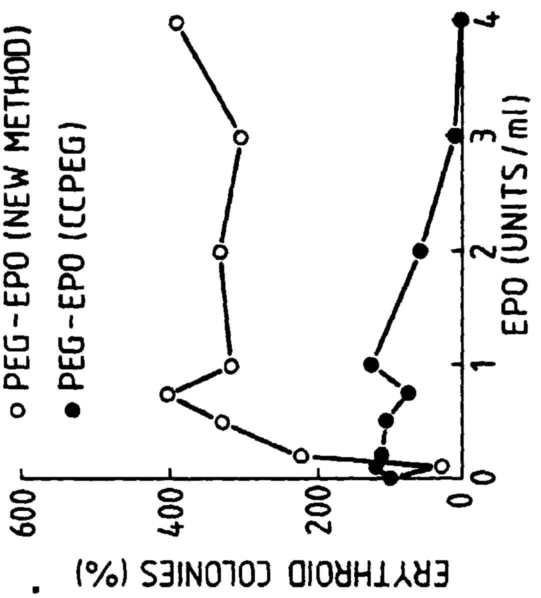


Fig.4b.

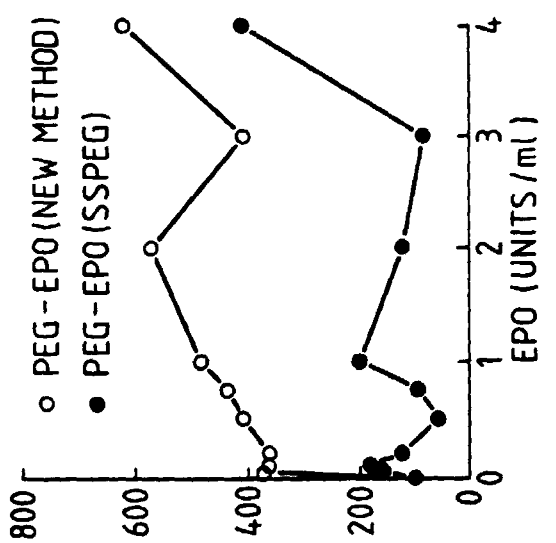


Fig.4c.

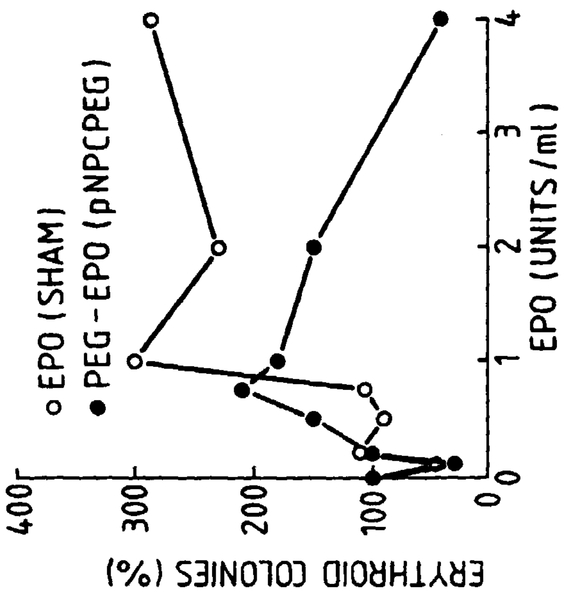


Fig.4d.

Fig.5.

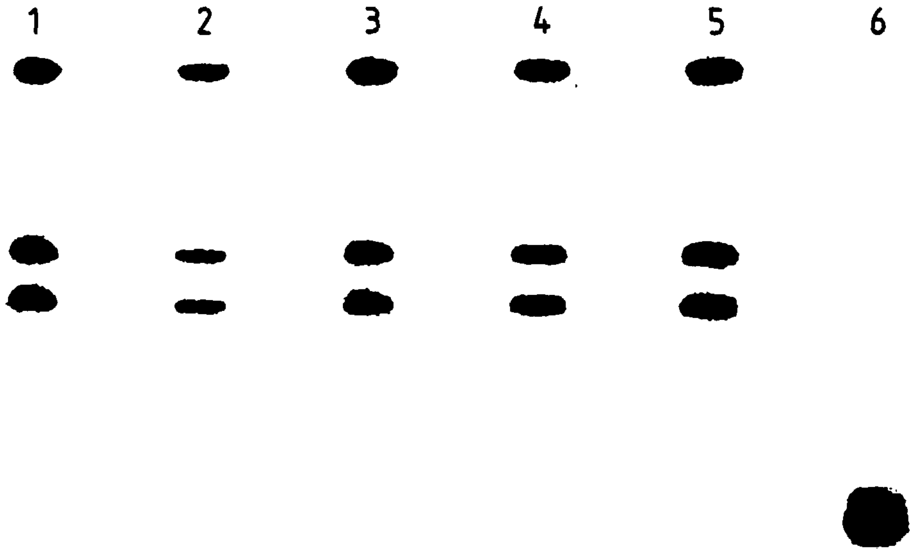
1
2
3
4
5
6

Fig.6a.

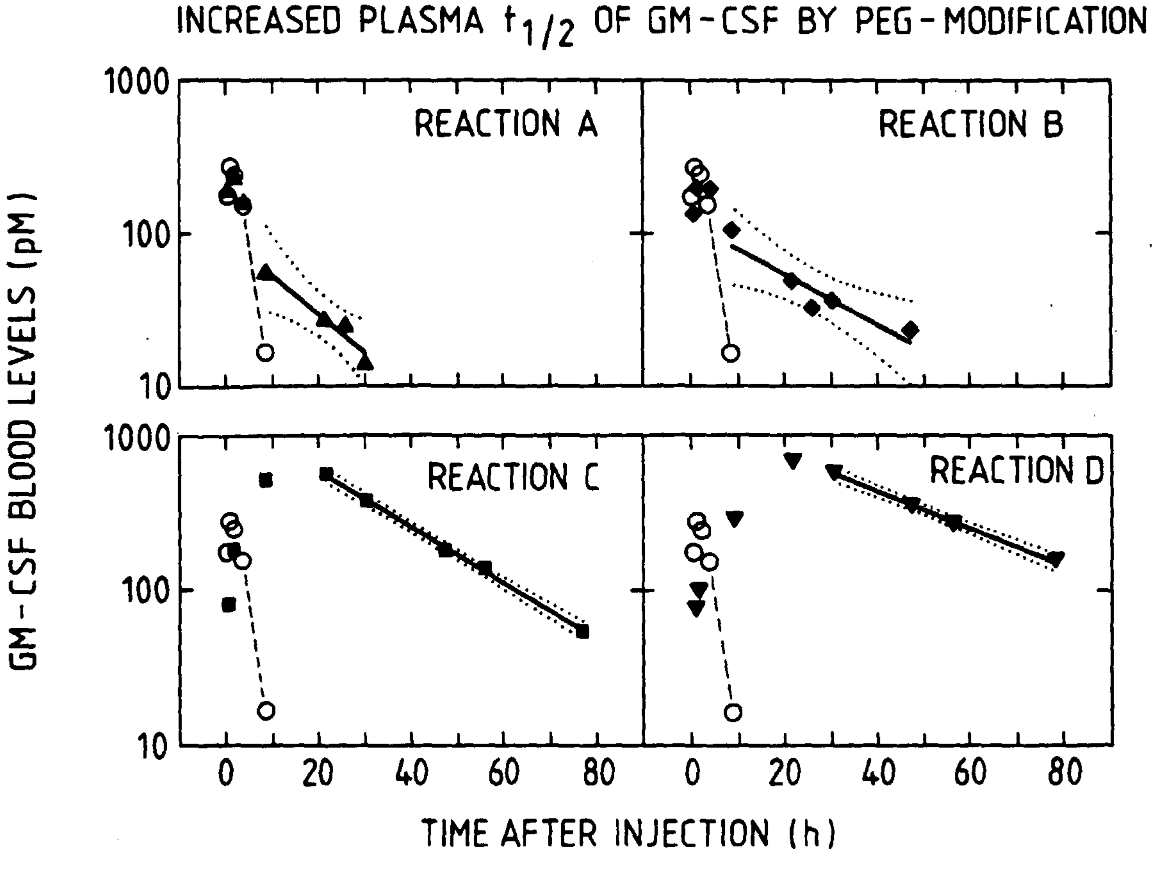
INCREASED PLASMA $t_{1/2}$ OF GM-CSF BY PEG-MODIFICATION
REACTION A
REACTION B
REACTION C
REACTION D
GM-CSF BLOOD LEVELS (pM)
1000
100
10
0
20
40
60
80
TIME AFTER INJECTION (h)

Fig.6b.

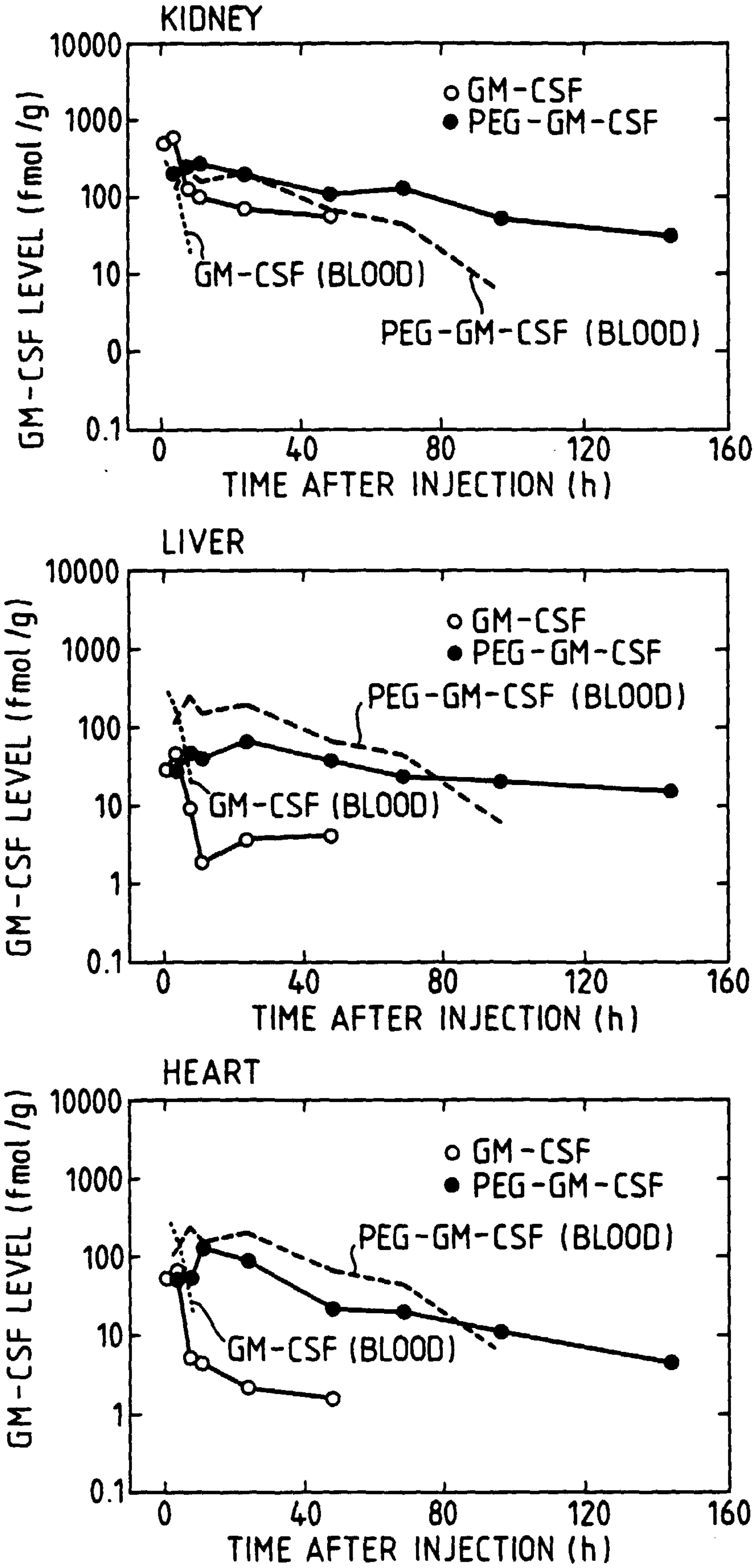
KIDNEY
GM-CSF LEVEL (fmol/g)
10000
1000
100
10
0
0.1
GM-CSF
PEG-GM-CSF
GM-CSF (BLOOD)
PEG-GM-CSF (BLOOD)
0
40
80
120
160
TIME AFTER INJECTION (h)
LIVER
GM-CSF LEVEL (fmol/g)
10000
1000
100
10
1
0.1
GM-CSF
PEG-GM-CSF
PEG-GM-CSF (BLOOD)
GM-CSF (BLOOD)
0
40
80
120
160
TIME AFTER INJECTION (h)
HEART
GM-CSF LEVEL (fmol/g)
10000
1000
100
10
1
0.1
GM-CSF
PEG-GM-CSF
PEG-GM-CSF (BLOOD)
GM-CSF (BLOOD)
0
40
80
120
160
TIME AFTER INJECTION (h)

Fig.6b(Cont).

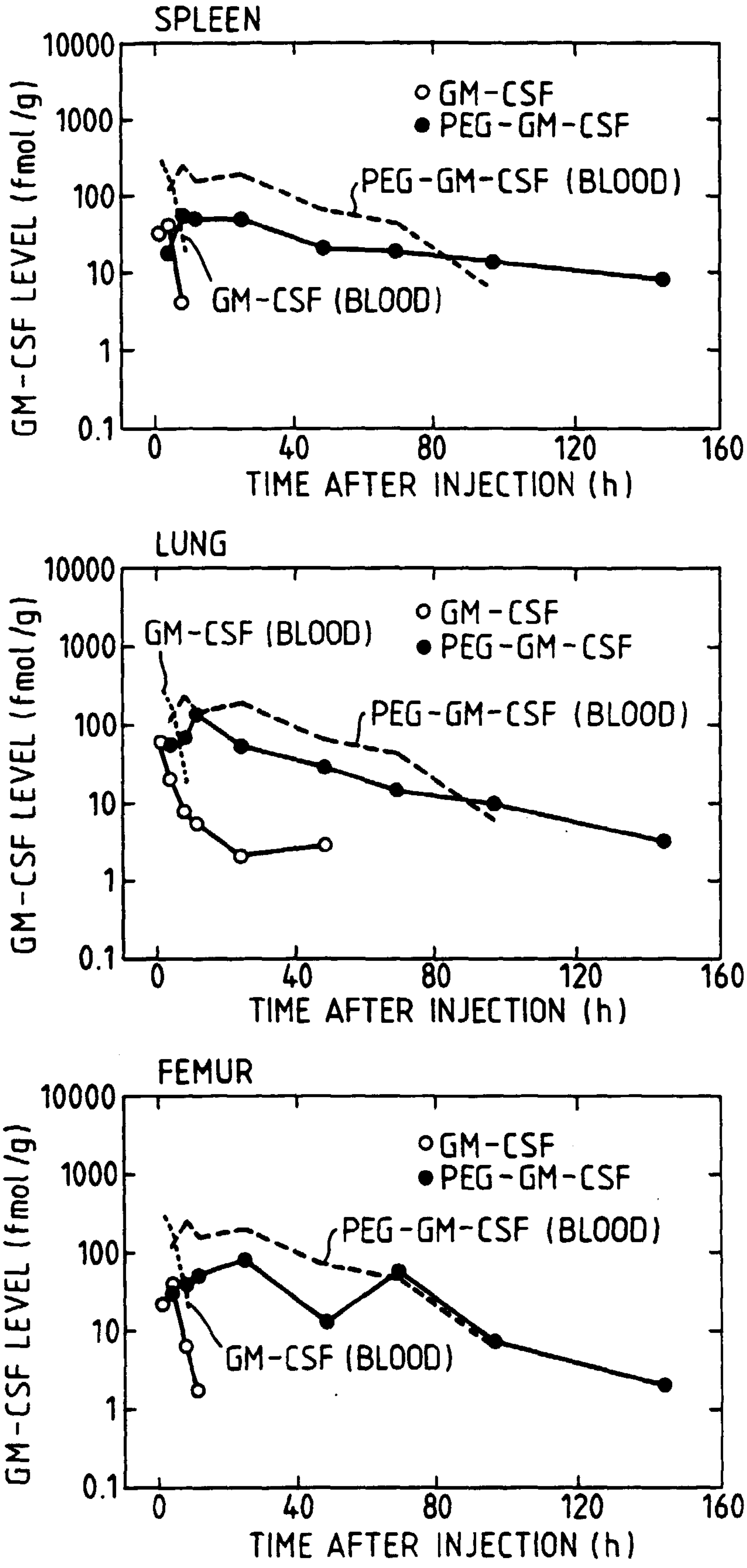
SPLEEN
GM-CSF LEVEL (fmol/g)
10000
1000
100
10
1
0.1
0
40
80
120
160
TIME AFTER INJECTION (h)
GM-CSF
PEG-GM-CSF
PEG-GM-CSF (BLOOD)
GM-CSF (BLOOD)
LUNG
GM-CSF LEVEL (fmol/g)
TIME AFTER INJECTION (h)
GM-CSF (BLOOD)
GM-CSF
PEG-GM-CSF
PEG-GM-CSF (BLOOD)
FEMUR
GM-CSF LEVEL (fmol/g)
TIME AFTER INJECTION (h)
GM-CSF
PEG-GM-CSF
PEG-GM-CSF (BLOOD)
GM-CSF (BLOOD)

Fig.7.

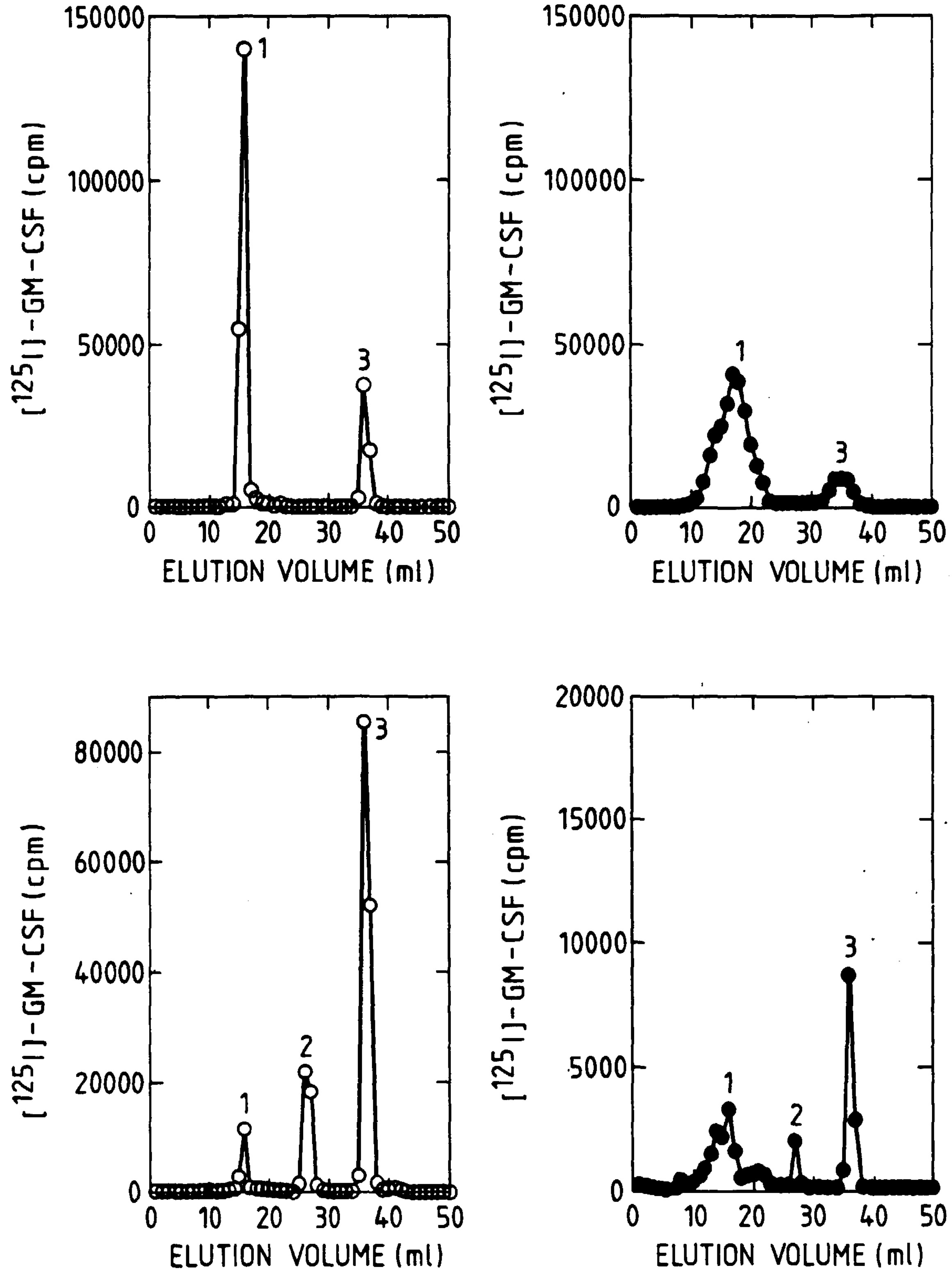
[125I]-GM-CSF (cpm)
ELUTION VOLUME (ml)
150000
100000
50000
80000
60000
40000
20000
15000
10000
5000
0
0
10
20
30
40
50
1
2
3

Fig.8a.

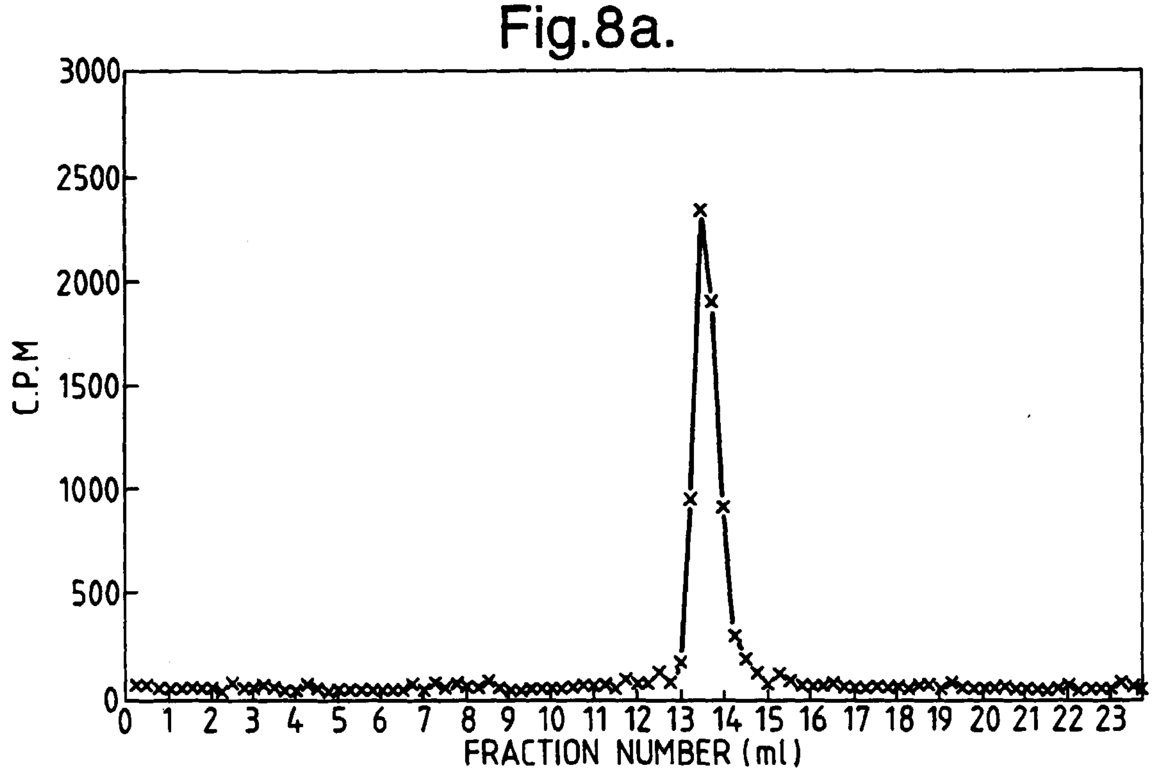
3000
2500
2000
1500
1000
500
0
C.P.M
0 1 2 3 4 5 6 7 8 9 10 11 12 13 14 15 16 17 18 19 20 21 22 23
FRACTION NUMBER (ml)

Fig.8b.

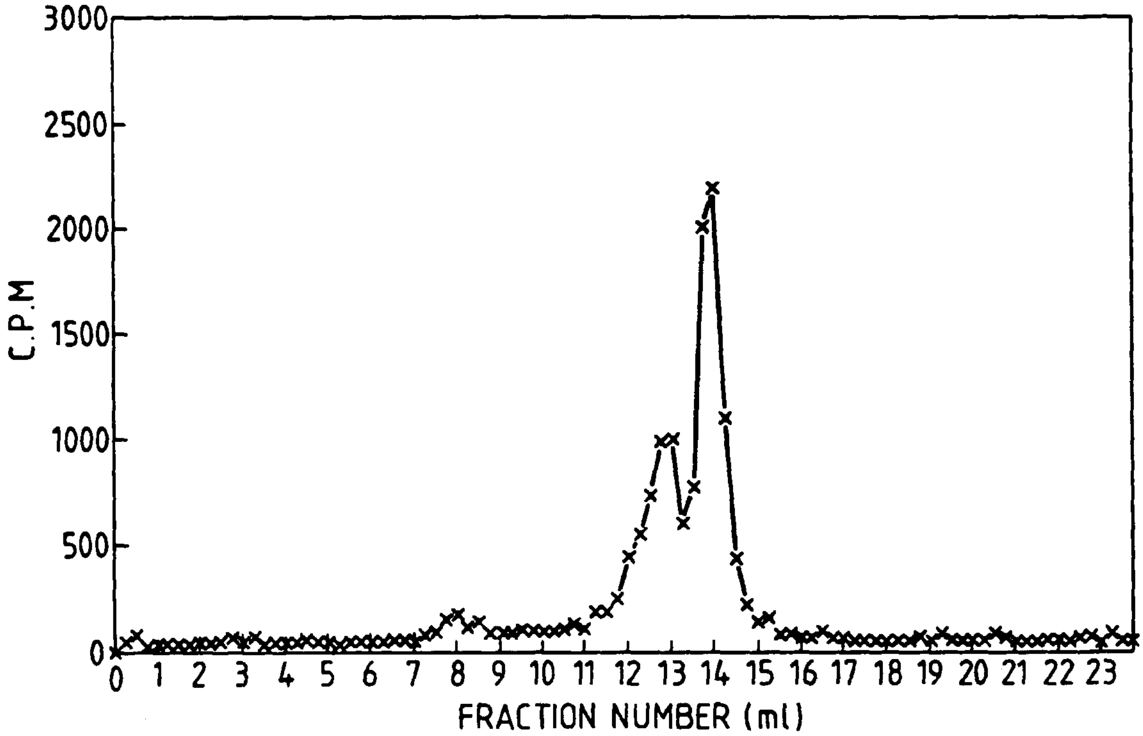
3000
2500
2000
1500
1000
500
0
C.P.M
0 1 2 3 4 5 6 7 8 9 10 11 12 13 14 15 16 17 18 19 20 21 22 23
FRACTION NUMBER (ml)

Fig.8c.

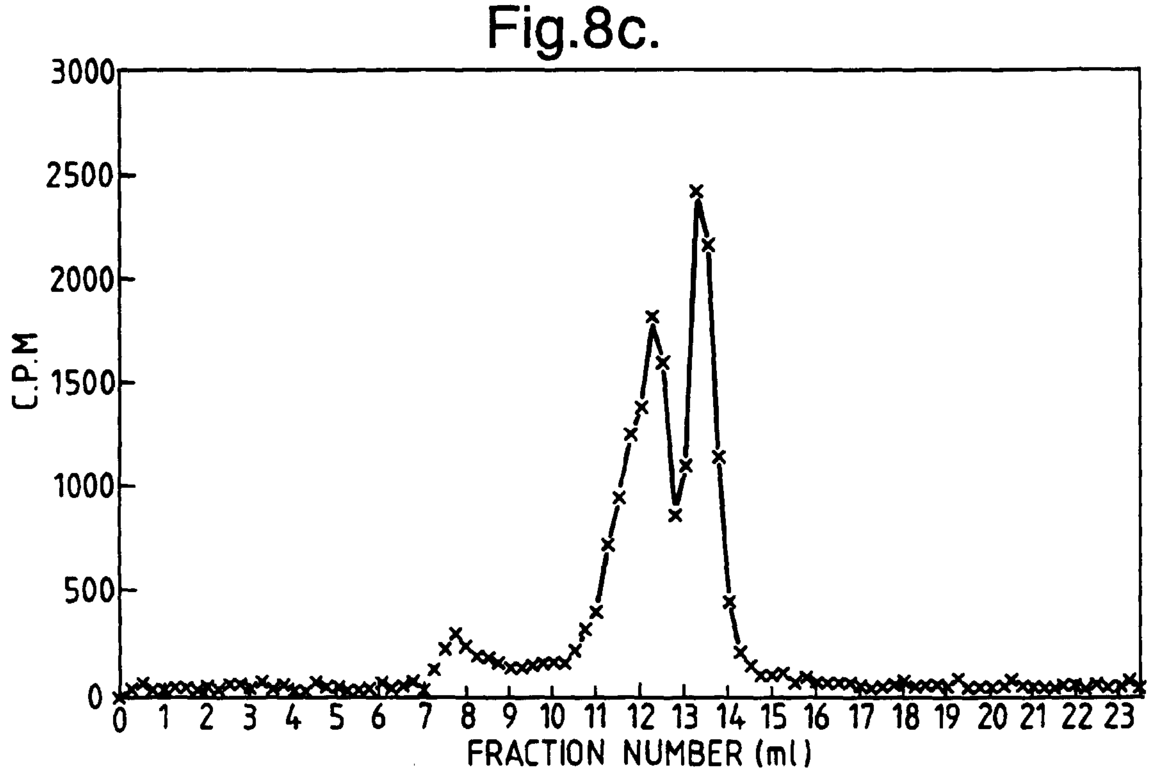
3000
2500
2000
1500
1000
500
0
C.P.M
0 1 2 3 4 5 6 7 8 9 10 11 12 13 14 15 16 17 18 19 20 21 22 23
FRACTION NUMBER (ml)

Fig.8d.

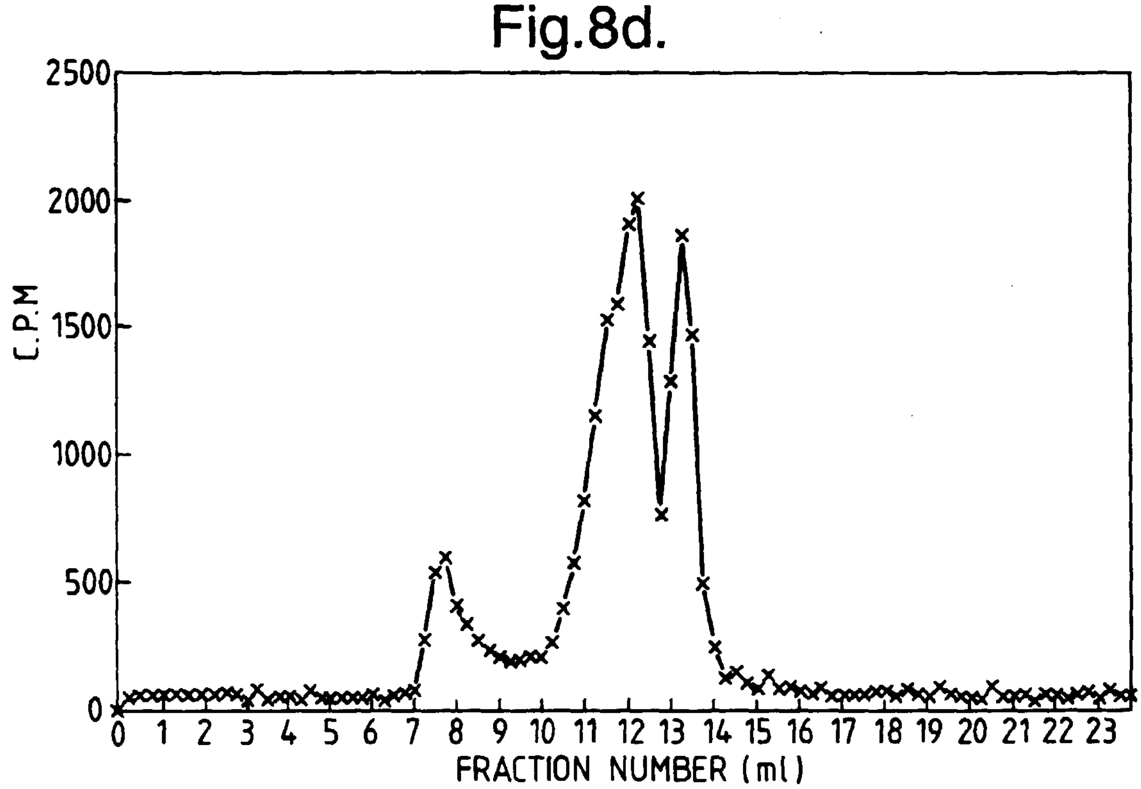
2500
2000
1500
1000
500
0
C.P.M
0 1 2 3 4 5 6 7 8 9 10 11 12 13 14 15 16 17 18 19 20 21 22 23
FRACTION NUMBER (ml)

Fig.8e.

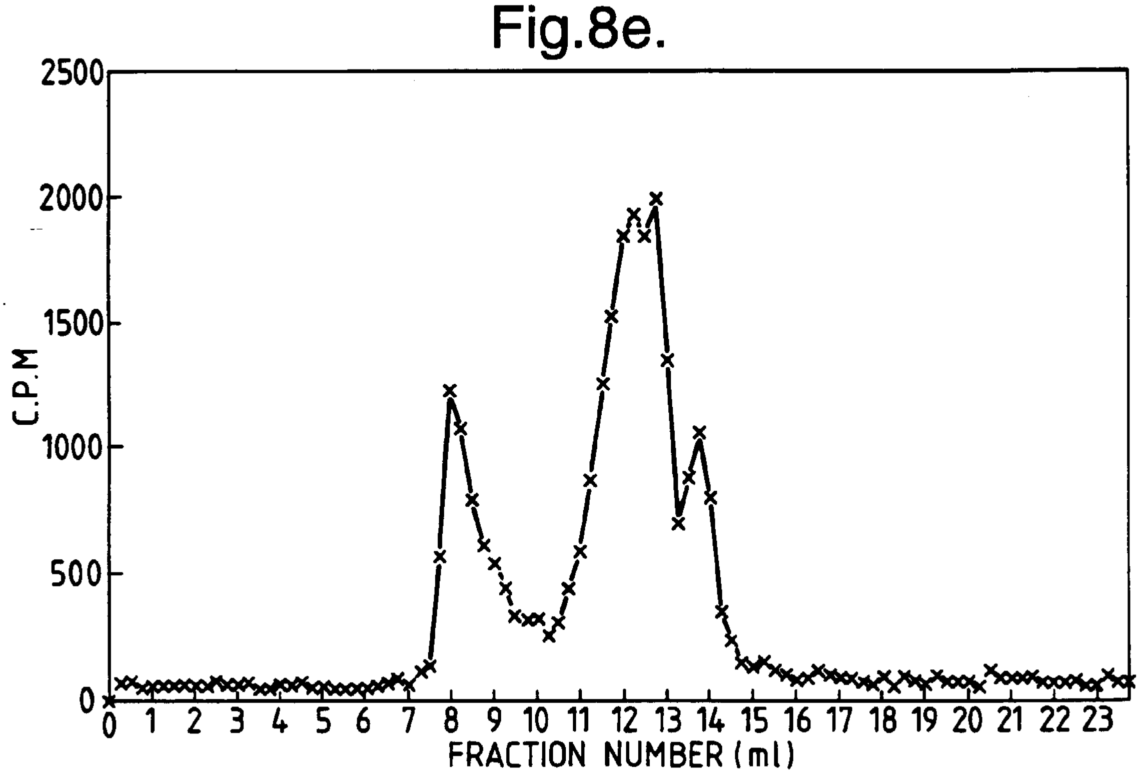
2500
2000
1500
1000
500
0
C.P.M
0 1 2 3 4 5 6 7 8 9 10 11 12 13 14 15 16 17 18 19 20 21 22 23
FRACTION NUMBER (ml)

Fig.8f.

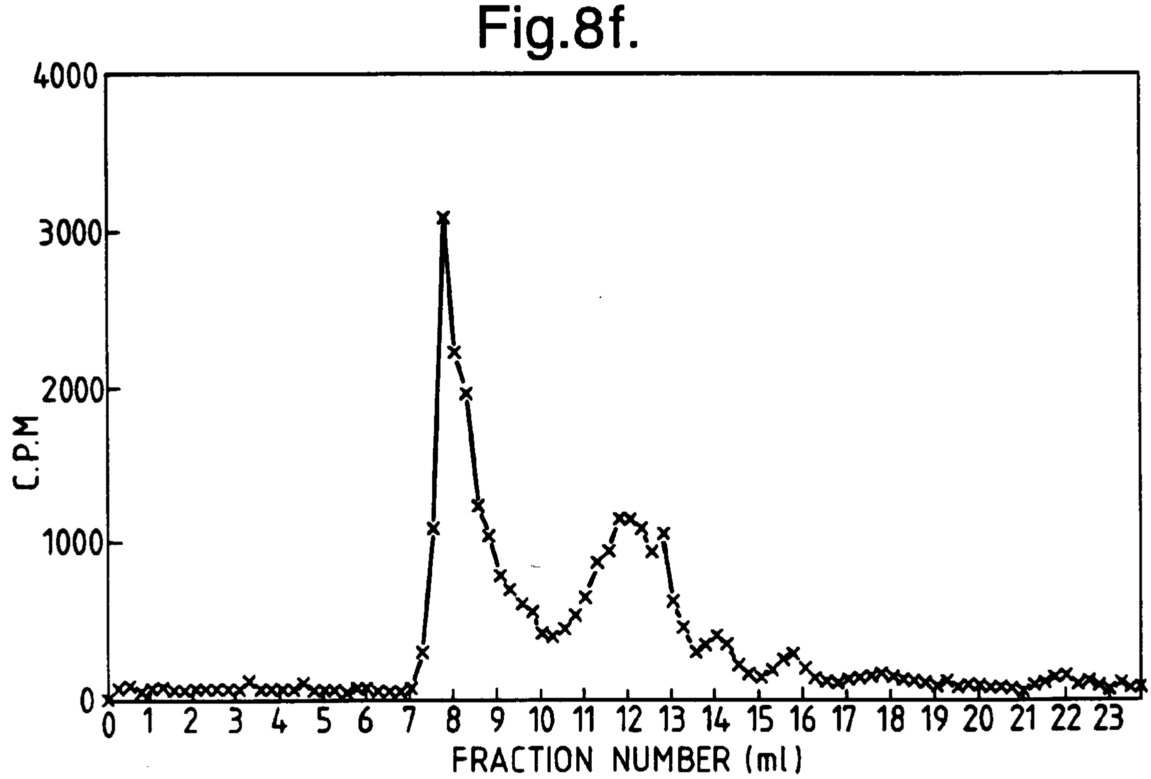
4000
3000
2000
1000
0
C.P.M
0 1 2 3 4 5 6 7 8 9 10 11 12 13 14 15 16 17 18 19 20 21 22 23
FRACTION NUMBER (ml)

Fig.9a.

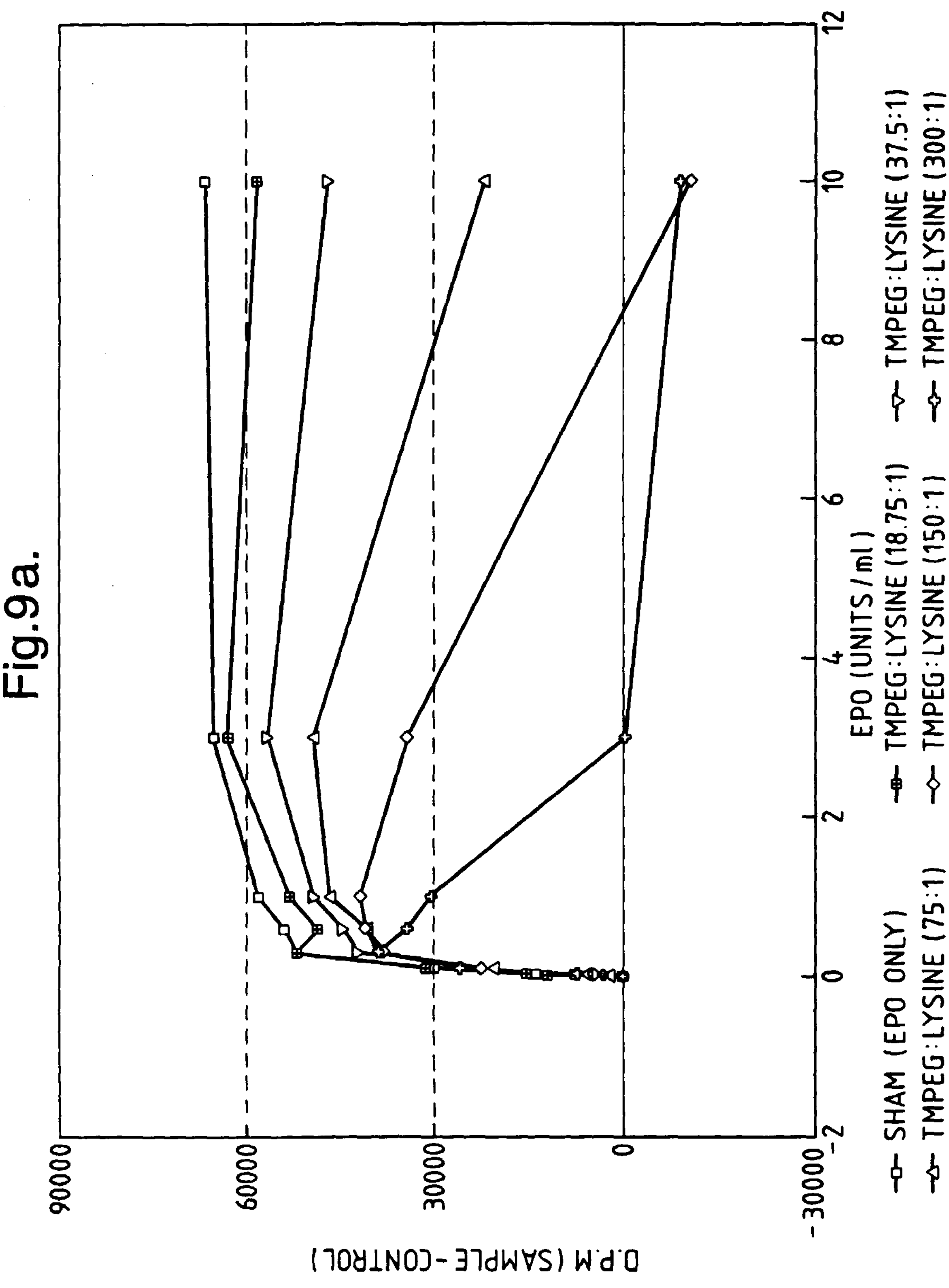
D.P.M (SAMPLE-CONTROL)
90000
60000
30000
0
-30000
-2
0
2
4
6
8
10
12
EPO (UNITS / ml)
SHAM (EPO ONLY)
TMPEG:LYSINE (18.75:1)
TMPEG:LYSINE (37.5:1)
TMPEG:LYSINE (75:1)
TMPEG:LYSINE (150:1)
TMPEG:LYSINE (300:1)

Fig.9b.

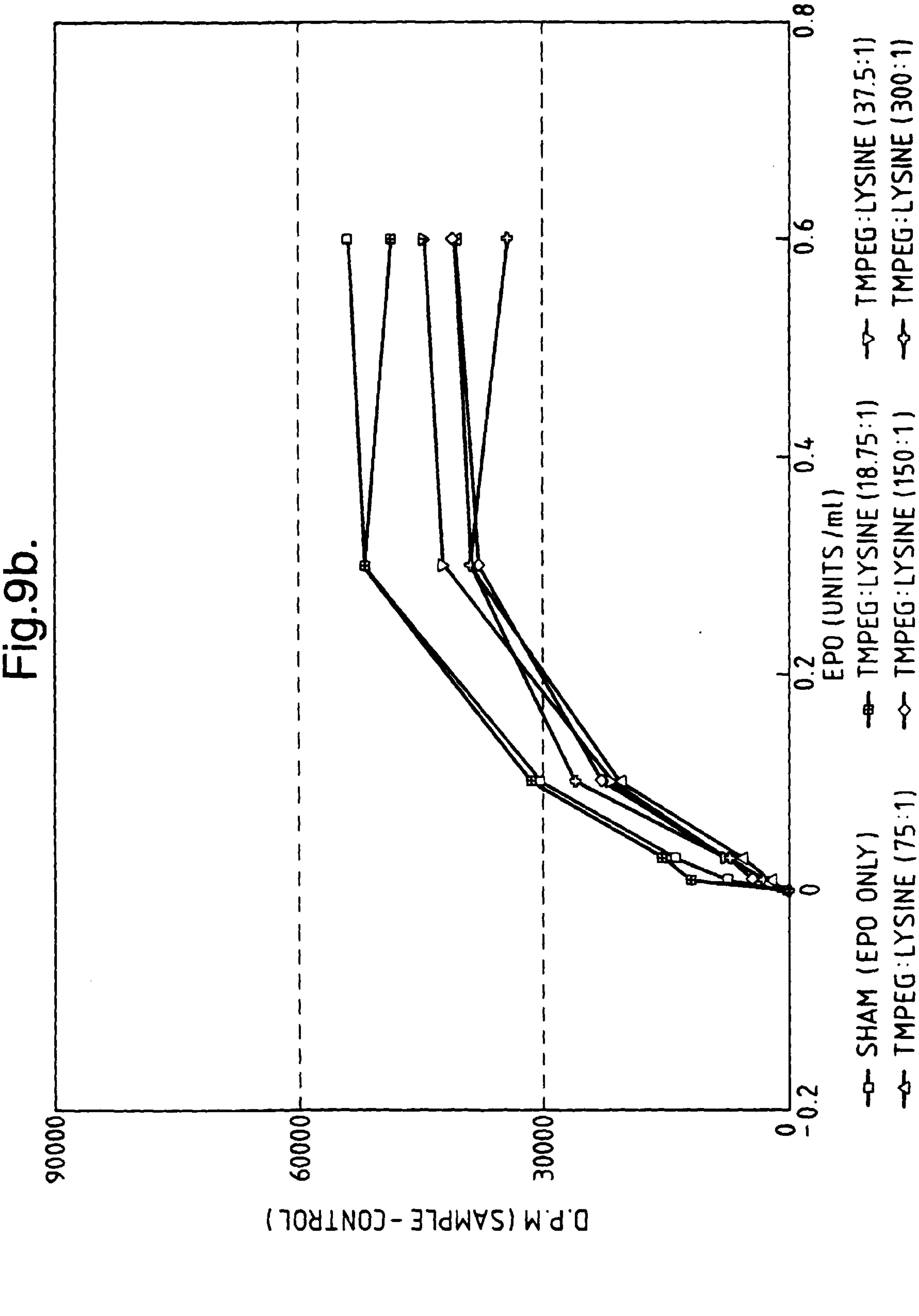
D.P.M (SAMPLE - CONTROL)
90000
60000
30000
0
-0.2
0
0.2
0.4
0.6
0.8
EPO (UNITS /ml)
SHAM (EPO ONLY)
TMPEG:LYSINE (18.75:1)
TMPEG:LYSINE (37.5:1)
TMPEG:LYSINE (75:1)
TMPEG:LYSINE (150:1)
TMPEG:LYSINE (300:1)